(19) Europäisches Patentamt
European Patent Office
Office européen des brevets

(11) EP 1 841 772 B1

(12) **EUROPÄISCHE PATENTSCHRIFT**

(45) Veröffentlichungstag und Bekanntmachung des Hinweises auf die Patenterteilung:
**06.05.2015 Patentblatt 2015/19**

(21) Anmeldenummer: **06700401.0**

(22) Anmeldetag: **05.01.2006**

(51) Int Cl.:
***C07D 491/044*** *(2006.01)* ***A61K 31/4355*** *(2006.01)*
***A61K 31/436*** *(2006.01)* ***A61P 19/00*** *(2006.01)*

(86) Internationale Anmeldenummer:
**PCT/EP2006/000047**

(87) Internationale Veröffentlichungsnummer:
**WO 2006/077013 (27.07.2006 Gazette 2006/30)**

(54) **TETRAHYDROFURANDERIVATE ALS INHIBITOREN VON MATRIX-METALLOPROTEINASEN**

TETRAHYDROFURANE DERIVATIVES FOR USE AS INHIBITORS OF MATRIX METALLOPROTEINASES

DERIVES DE TETRAHYDROFURANE EN TANT QU'INHIBITEURS DE METALLOPROTEINASES MATRICIELLES

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **19.01.2005 DE 102005002500**

(43) Veröffentlichungstag der Anmeldung:
**10.10.2007 Patentblatt 2007/41**

(73) Patentinhaber: **SANOFI**
**75008 Paris (FR)**

(72) Erfinder:
- **SCHUDOK, Manfred**
  **65817 Eppstein/Ts. (DE)**
- **MATTER, Hans**
  **63505 Langenselbold (DE)**
- **HOFMEISTER, Armin**
  **55278 Dexheim (DE)**

(74) Vertreter: **Then, Johann**
**Sanofi-Aventis Deutschland GmbH**
**Global Intellectual Property Department**
**Industriepark Höchst**
**Gebäude K 703**
**65926 Frankfurt am Main (DE)**

(56) Entgegenhaltungen:

| | |
|---|---|
| **EP-A- 0 803 505** | **EP-A- 1 065 209** |
| **EP-A- 1 217 002** | **WO-A-97/18194** |
| **WO-A-99/06410** | **WO-A-03/016248** |
| **WO-A-2005/030728** | **WO-A-2006/002764** |

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Die Erfindung betrifft neue Derivate bicylischer Tetrahydrofuran-Iminosäuren, Verfahren zu ihrer Herstellung und Verwendung derselben als Arzneimittel.

In Erkrankungen wie Osteoarthritis und Rheuma findet eine Zerstörung des Gelenkes statt, besonders bedingt durch den proteolytischen Abbau von Kollagen durch Kollagenasen. Kollagenasen gehören zur Superfamilie der Metalloproteinasen (MP) bzw. Matrix-Metallproteinasen (MMP's). Die MMP's bilden eine Gruppe von Zn-abhängigen Enzymen, die am biologischen Abbau der extrazellulären Matrix beteiligt sind (D. Yip et al. in Investigational New Drugs 17 (1999), 387-399 und Michaelides et al. in Current Pharmaceutical Design 5 (1999) 787 -819). Diese MMP's sind insbesondere fähig, fibrilläres und nicht-fibrilläres Kollagen, sowie Proteoglycane abzubauen, die beide wichtige Matrixbestandteile darstellen. MMP's sind beteiligt an Prozessen der Wundheilung, der Tumorinvasion, Metastasenwanderung sowie an Angiogenese, multipler Sklerose und Herzversagen (Michaelides Seite 788; siehe oben). Insbesondere spielen sie eine wichtige Rolle beim Abbau der Gelenkmatrix in der Arthrose und der Arthritis, sei es nun die Osteoarthrose, Osteoarthritis oder die rheumatoide Arthritis.

Die Aktivität der MMP's ist weiterhin essentiell für viele der Prozesse, die bei der atherosklerotischen Plaque-Bildung eine Rolle spielen, wie die Infiltration inflammatorischer Zellen, der glatten Muskelzell-Migration sowie Proliferation und Angiogenese (S. J. George, Exp. Opin. Invest. Drugs (2000), 9 (5), 993-1007). Weiterhin kann die Matrix-Degradation durch MMPs Plaque-Instabilitäten bis hin zu Rupturen verursachen, was zu den klinischen Symptomen der Atherosklerose, instabilen Angina Pectoris, Myokardinfarkt oder Schlaganfall führen kann (E. J. M. Creemers et al, Circulation Res. 89,201-210 (2001)). Insgesamt betrachtet kann die gesamte MMP-Familie alle Komponenten der extrazellulären Matrix der Blutgefäße abbauen; deshalb ist deren Aktivität in starkem Maße Regulationsmechanismen in normalen Blutgefäßen unterworfen. Die erhöhte MMP-Aktivität während der Plaque-Bildung und Plaque-Instabilität wird durch erhöhte Cytokin- und Growth-Faktor-stimulierte Gen-Transkription, erhöhte Zymogen-Aktivivierung und eine Imbalance des MMP-TIMP-Verhältnisses verursacht (Tissue inhibitors of metalloproteases). Deshalb erscheint es einleuchtend, dass eine MMP-Inhibierung oder die Wiedererlangung der MMP-TIMP-Balance hilfreich sein wird in der Behandlung der atherosklerotischen Erkrankungen. Ebenso wird es immer deutlicher, dass neben der Atherosklerose auch weitere cardiovaskuläre Erkrankungen durch eine erhöhte MMP-Aktivität zumindest mitverursacht werden, wie beispielsweise Restenose, dilatierte Cardiomyopathie und der schon erwähnte Myokardinfarkt. Es konnte gezeigt werden, dass durch die Applikation synthetischer Inhibitoren in experimentellen Tiermodellen dieser Erkrankungen deutliche Verbesserungen erzielt werden konnten, z. B. betreffend Bildung atherosklerotischer Läsionen, Neointima-Bildung, Linksventrikuläres Remodelling, Dysfunktion der Pumpleistung oder Infarkt-Heilung. In verschiedenen präklinischen Studien mit MMP-Inhibitoren zeigte sich bei detaillierter Gewebsanalyser eine reduzierte Kollagen-Schädigung, verbessertes extrazelluläres Matrix-Remodelling und verbesserte Struktrur und Funktion von Herzmuskel und Gefäßen. Von diesen Prozessen werden insbesondere die Matrix-Remodelling-Prozesse und MMP-regulierte Fibrosen als wichtige Komponenten im Fortschreiten von Herzerkrankungen (Infarkt) angesehen (Drugs 61, 1239-1252 (2001)).

MMP's spalten Matrixproteine wie Kollagen, Laminin, Proteoglykane, Elastin oder Gelatin sowie prozessieren (d. h. aktivieren oder desaktivieren) durch eine Spaltung eine Vielzahl weiterer Proteine und Enzyme unter physiologischen Bedingungen, so daß Ihnen eine wichtige Rolle im gesamten Organismus zukommt, mit besonderer Bedeutung im Bindegewebe und Knochen.

Eine Vielzahl verschiedener Inhibitoren der MMP's sind bekannt (EP 0 606 046; WO 94/28889; WO 96/27583; oder auch Übersichten wie Current Medicinal Chemistry 8, 425-74 (2001), Current Medicinal Chemistry 11, 2911-2977 (2004) oder Current Opinion in Drug Discovery & Development 7, 513-535 (2004). Nach den ersten klinischen Studien an Menschen hat sich nun gezeigt, dass MMP's Nebenwirkungen hervorrufen. Die hauptsächlich genannten Nebenwirkungen sind muskuloskeletale Schmerzen oder Anthralgien. Der Stand der Technik besagt eindeutig, dass selektivere Inhibitoren diese genannten Nebenwirkungen reduzieren können (Yip, Seite 387, siehe oben). Besonders hervorzuheben ist dabei eine Spezifität gegenüber MMP-1, da mit der Hemmung von MMP-1 offensichtlich die unerwünschten Nebenwirkungen verstärkt auftreten.

Nachteil der bekannten Inhibitoren der MMP's sind daher häufig die mangelnde Spezifität. Die meisten MMP-Inhibitoren hemmen viele MMP's gleichzeitig, weil die katalytische Domäne der MMP's eine ähnliche Struktur aufweist. Demzufolge wirken die Inhibitoren in unerwünschter Weise auf die Enzyme, auch solche mit vitaler Funktion, ein (Massova I, et al., The FASEB Journal (1998) 12, 1075-1095).

Strukturell betrachtet lassen sich die meisten Matrix-Metalloproteinase-Inhibitoren in Sulfonamide und Sulfone einteilen, die eine Zink-bindende Gruppe tragen. Besonders bevorzugt ist dabei die Carbonsäure- und ganz besonders die Hydroxamsäure-Gruppe. Die Eigenschaften sind im Detail etwa in den oben angeführten Übersichtsartikeln beschrieben. Die Gruppe der Sulfonamide ist dadurch gekennzeichnet, dass als strukturelle Basis oft ein Amino- oder Iminocarbonsäure-Grundgerüst genutzt wird. Auch werden bicyclische Iminosäuregrundgerüste eingesetzt, besonders in Kombination mit phenylischen Ringsystemen. Dagegen sind bislang nur vergleichsweise wenige heterobicyclische Iminosäure-Grundgerüste in MMP-Inhibitoren zu finden, insbesondere, wenn man sauerstoffhaltige Heteroaryle, also

Furane, betrachtet. Diese bicyclischen Furansysteme wurden beispielsweise in EP 0803505, EP 1065209, EP 1217002 oder in WO 99/06410 beschrieben und werden auch in ähnlicher Form in PCT/US02/26018 offenbart.

In dem Bestreben, wirksame Verbindungen zur Behandlung von Bindegewebserkrankungen zu finden, wurde nun gefunden, dass die erfindungsgemäß eingesetzten Derivate starke Inhibitoren der Matrix-Metalloproteinasen MMP-2, MMP-3 MMP-8, MMP-9 und MMP-13 sind, gleichzeitig aber MMP-1, das möglicherweise für die Nebeneffekte verantwortlich ist, wesentlich weniger gehemmt wird.

Die Erfindung betrifft daher eine Verbindung der Formel II,

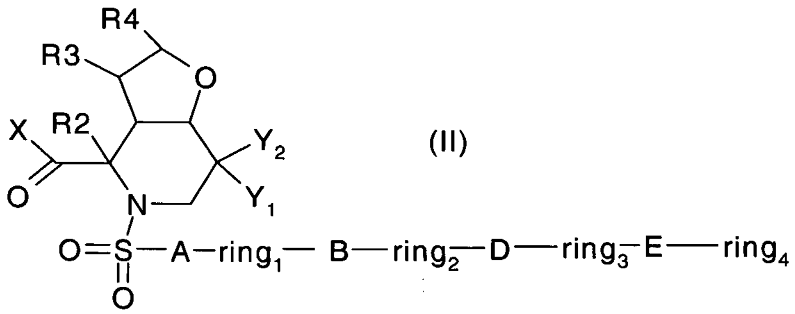

(II)

wobei

A für eine kovalente Bindung steht,
B, D und E gleich oder verschieden sind und unabhängig voneinander für eine kovalente Bindung oder den Rest -O- stehen,
ring1, ring2 oder ring3 gleich oder verschieden sind und unabhängig voneinander für

1) kovalente Bindung stehen oder
2) Phenyl bedeutet und unsubstituiert oder unabhängig voneinander ein- oder zweifach durch G substituiert sind,

mit der Maßgabe, dass mindestens einer der Reste ring1, ring2 oder ring3 für Phenyl steht,
ring4 für Phenyl steht und unsubstituiert oder unabhängig voneinander ein- oder zweifach durch G substituiert ist,
G für 1) Wasserstoffatom,

2) Br, Cl oder F,
3) -($C_1$-$C_4$)-Alkyl, worin Alkyl unsubstituiert oder ein-, zwei- oder dreifach durch Br, Cl oder F substituiert ist,
4) -$SO_2$-Methyl oder
5) -CN steht,

X für -OH oder -NH-OH steht, und
R2, R3, R4, Y1 und Y2 gleich sind und für Wasserstoffatom stehen.

Ein weiterer Gegenstand der Erfindung ist die Verbindung der Formel II aus der Reihe
5-(4'-Chlorbiphenyl-4-sulfonyl)-octahydrofuro[3,2-c]pyridin-4-carbonsäure,
5-(4'-Chlorbiphenyl-4-sulfonyl)-octahydrofuro[3,2-c]pyridin-4-(N-hydroxy)-carboxamid,
5-[4-(4-Fluorphenoxy)-benzolsulfonyl]-octahydro-furo[3,2-c]pyridin-4-carbonsäure,
5-[4-(4-Fluorphenoxy)-benzolsulfonyl]-octahydro-furo[3,2-c]pyridin-4-(N-hydroxy)-carboxamid,
5-[4-(4-Cyanophenoxy)-benzolsulfonyl]-octahydro-furo[3,2-c]pyridin-4-carbonsäure,
5-[4-(4-Methansulfonylphenoxy)-benzolsulfonyl]-octahydro-furo[3,2-c]pyridin-4-carbonsäure,
5-(4'-Fluorbiphenyl-4-sulfonyl)-octahydro-furo[3,2-c]pyridin-4-(N-hydroxy)-carboxamid,
5-(4'-Trifluormethylbiphenyl-4-sulfonyl)-octahydro-furo[3,2-c]pyridin-4-(N-hydroxy)-carboxamid,
5-[4-(4-Chlorphenoxy)-benzolsulfonyl]-octahydro-furo[3,2-c]pyridin-4-(N-hydroxy)-carboxamid,
5-[4-(4-Cyanophenoxy)-benzolsulfonyl]-octahydro-furo[3,2-c]pyridin-4-(N-hydroxy)-carboxamid oder
5-[4-(4-Methansulfonylphenoxy)-benzolsulfonyl]-octahydro-furo[3,2-c]pyridin-4-(N-hydroxy) carboxamid.

Unter dem Begriff "($C_1$-$C_6$)-Alkyr werden Kohlenwasserstoffreste verstanden, deren Kohlenstoffkette geradkettig oder verzweigt ist und 1 bis 6 Kohlenstoffatome enthält, beispielsweise Methyl, Ethyl, Propyl, Iso-Propyl, Butyl, Iso-Butyl, tertiär-Butyl, Pentyl, Iso-Pentyl, Neopentyl, Hexyl, 2,3-Dimethylbutan oder Neohexyl.

Unter dem Begriff "-($C_0$-$C_4$)-Alkylen" werden Kohlenwasserstoffreste verstanden, deren Kohlenstoffkette geradkettig oder verzweigt ist und 1 bis 4 Kohlenstoffatome enthält, beispielsweise Methylen, Ethylen, Propylen, Iso-Propylen, Iso-Butylen, Butylen oder tertiär-Butylen. "-$C_0$-Alkylen" ist eine kovalente Bindung.

Wenn mehrere der Reste A, B, D, E, ring1, ring2 oder ring3 hintereinander für jeweils eine kovalente Bindung stehen sollten, so bleibt immer nur eine kovalente Bindung übrig und die anderen kovalenten Bindungen entfallen. Sollte beispielsweise A und ring1 jeweils eine kovalente Bindung darstellen, so entfällt eine kovalente Bindung und nur eine kovalente Bindung bleibt übrig. Sollten beispielsweise B, ring2, D und ring3 jeweils eine kovalente Bindung darstellen, so entfallen drei kovalente Bindungen und nur eine kovalente Bindung bleibt übrig.

Die Erfindung betrifft ferner ein Verfahren zur Herstellung der Verbindung der Formel II und/oder einer stereoisomeren Form der Verbindung der Formel II und/oder eines physiologisch verträglichen Salzes der Verbindung der Formel II, das dadurch gekennzeichnet ist, dass man

a) eine Verbindung der Formel IV,

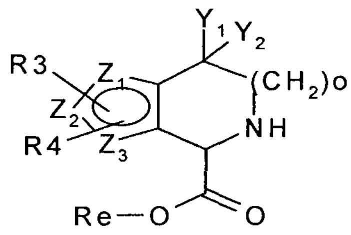

(IV)

worin Re ein Wasserstoffatom oder eine Ester-Schutzgruppe darstellt, die Reste Y1, Y2, R3, R4 und o wie in der Verbindung der Formel II definiert sind und die Teilstruktur der Verbindung der Formel II

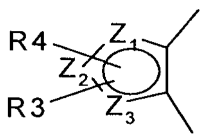

ein ungesättigter Ring mit 5 Ringatomen ist, wobei eines der Ringatome Z1, Z2 oder Z3 für ein Sauerstoffatom steht und die beiden anderen Ringatome für Kohlenstoffatome stehen, die unabhängig voneinander durch R3 oder R4 substituiert sind,

durch Hydrierung unter geeigneten Bedingungen zu einer Verbindung der Formel V umwandelt,

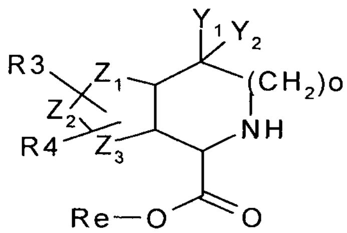

(V)

worin die Teilstruktur der Verbindung der Formel II

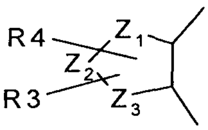

ein gesättigter Ring mit 5 Ringatomen ist, wobei eines der Ringatome Z1, Z2 oder Z3 für ein Sauerstoffatom steht und die beiden anderen Ringatome für Kohlenstoffatome stehen, die unabhängig voneinander durch R3 oder R4 substituiert sind, und die Reste Y1, Y2, R3, R4 und o wie in der Verbindung der Formel IV definiert sind,

b) anschließend die Verbindung der Formel V mit einer Verbindung der Formel VI,

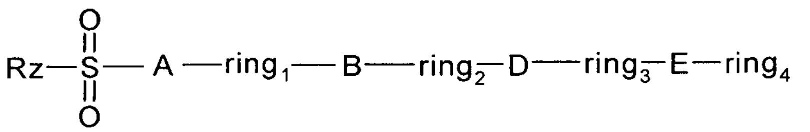

(VI)

worin A, B, D, E und ring1, ring2, ring3, ring4 wie in Formel II definiert sind, und worin Rz Chloratom, Bromatom, Imidazoyl oder OH bedeutet,
in Gegenwart einer Base oder nach Silylierung mit einem geeigneten Silylierungsmittel oder mit einem geeigneten wasserentziehenden Mittel für den Fall Rz = OH zu einer Verbindung der Formel VII umsetzt,

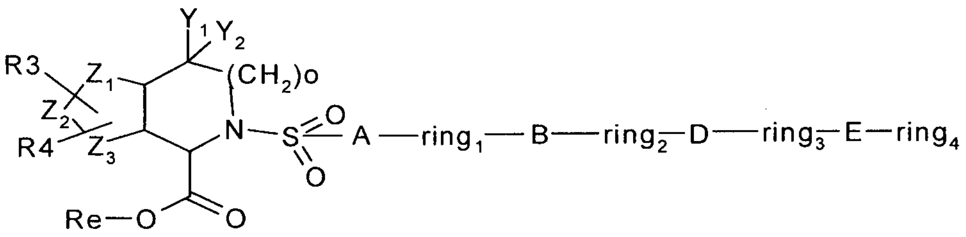

(VII)

worin A, B, D, E, Re und ring1, ring2, ring3 und ring4 wie oben definiert sind, und
b) für den Fall Re = Ester eine nach a) hergestellte Verbindung der Formel VII mit einer Alkalilauge wie NaOH oder LiOH und anschließender Säurebehandlung zu der erfindungsgemäßen Carbonsäure der Formel II, worin X = OH ist, umsetzt, wobei gegebenenfalls vorher noch Modifikationen in einer der Seitenketten der Ringe ring1-ring4 vorgenommen wurden; oder den genannten Ester durch Behandlung mit Mineralsäure wie Salzsäure zur freien Carbonsäure der Formel VIII umsetzt

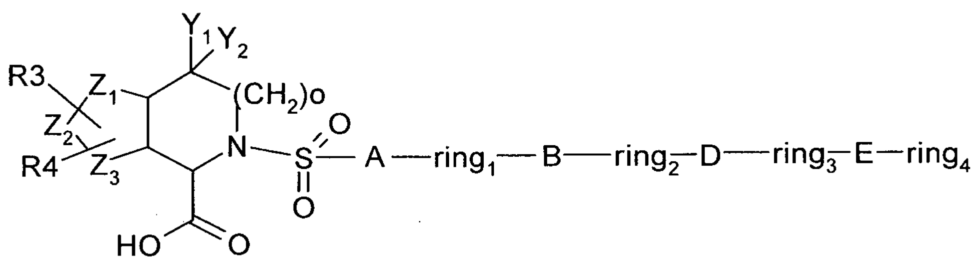

(VIII)

oder anschließend die Verbindung der Formel VIII in die Hydroxamsäure, worin X = NH-OH ist, der Formel II umwandelt,
c) eine nach Verfahren a) hergestellte Verbindung der Formel II, oder eine geeignete Vorstufe der Formel II, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder
d) die nach den Verfahren b) oder c) hergestellte Verbindung der Formel II entweder in freier Form isoliert oder im Falle des Vorliegens von sauren oder basischen Gruppen in physiologisch verträgliche Salze umwandelt.

Verbindungen der Art der Formeln IV bis VIII stellen bizyklische Verbindungen dar, die einen funktionalisierten Rings mit sechs Ringatomen oder einen Ring mit sieben Ringatomen enthalten. Der zweite Ring ist ein ungesättigtes Furan (Formel IV) oder ein gesättigtes, heterocyclisches Tetrahydrofuran-System, wobei das SauerstoffAtom entsprechend der Formel II an jeder der drei möglichen Positionen stehen kann. Beide Ringe können entsprechend der Formel II auch substituiert sein.

Verbindungen des Typs der Formel IV lassen sich nach bekannten Vorschriften herstellen. Beispielsweise können Verbindungen mit o = 1 nach bekannten Verfahren aus den entsprechenden Furan-Derivaten hergestellt werden. Die Herstellung dieser Verbindungen ist dem Fachmann bekannt und kann nach verschiedenen Verfahren erfolgen. Die Synthese von Furanen ist beispielsweise in: Science of Synthesis 9, 183-285 (2002), beschrieben worden. Eine bewährte Methode zur Herstellung der bicyclischen Ausgangsverbindungen geht beispielsweise von den 2-Aminoethyl- oder 3-Aminopropyl-(2- oder 3- furyl)-Derivaten aus. In einer Pictet-Spengler-artigen Reaktion erfolgt Cyclisierung unter sauren

Bedingungen mit Glyoxylsäuren oder deren Estern. Diese Reaktion ist beispielsweise in J. Med. Chem. 37, 2138-2144 (1994) beschrieben. Aber auch andere Methoden zum Aufbau des zu den erfindungsgemäßen Tetrahydrofuranen führenden analogen Furan-Systemen sind anwendbar. Insbesondere in Abhängigkeit der Substituenten oder Ringgröße wird der Fachmann die geeigneten Synthesen auswählen. Nach Synthese der Furanen erfolgt Umwandlung zu den Tetrahydrofuranen. In der Regel erfolgt diese durch katalytische Hydrierung. In der Literatur sind eine Vielzahl von Verfahren beschrieben. Die Auswahl der geeigneten Bedingungen richtet sich nach der Reaktivität des Grundkörpers, eventuell vorhandenen funktionellen Gruppen oder Substituenten und inwieweit durch die Verwendung chiraler Hilfstoffe und Katalysatoren auch chirale Verbindungen erzeugt werden sollen. So werden folgende Katalysatoren und Reagenzien zur Hydrierung häufiger beschrieben, es ist jeweils nur eine Literaturstelle beispielhaft genannt: H2, Pd/C (z. B. in Arch. Pharm. 336, 381-4 (2003) oder Synthesis 2004, S. 2069-2077); auch als Transfer-Hydrierung mit Ammoniumformiat: Heterocycles 35, 737-754 (1993)), Na in flüssigem Ammoniak (z. B. J. Heterocycl. Chem. 37, 751-55 (2000)); Raney-Nickel (Synth. Commun. 25, 2895-2900 (1995)) oder Ni auf Trägermaterialien (J. Mol. Catal. 57, 397 (1990); J. Heterocycl. Chem. 3, 101 (1966)); Rh auf Trägermaterialien (J. Org. Chem. 37, 4260 (1972)); PdO (Org. Synth. 1943, II, 566)); LiAlH4 kann ebenfalls eingesetzt werden (J. Chem. Soc. 1957, S. 1788). Ebenso findet man Methoden zur enantioselektiven homogenen Hydrierung mit speziellen Rh-Katalysatoren (Monatsh. Chem. 131, 1335-1343 (2000), oder enantiodifferenzierende Hydrierung mit speziell modifziertem Raney-Nickel (Chem. Lett. 1999, S. 1055-56). Furan-Derivate, die noch Substituenten Y1 und Y2 tragen, sind auch nach anderen Verfahren zugänglich. Beispielsweise findet sich eine analoge Synthese der Verbindung mit Y1 und Y2 gleich C=O in WO2002/100860. Verbindungen dieses Typs stellen wichtige Ausgangsprodukte dar und lassen sich nach einer Vielzahl von Methoden, die dem Fachmann bekannt sind, in Verbindungen mit anderen Substituenten Y1 und Y2 umwandeln. Auch in diesem Fall erfolgt auf geeigneter Stufe die Umwandlung des Furans in das Tetrahydrofuran zu Verbindungen der Formel II.

Tetrahydrofuran-Synthesen wurden bereits beschrieben und sind dem Fachmann bekannt. Durch geeignete Wahl von Ausgangsprodukten und Substituenten können die erfindungsgemäßen Verbindungen ebenfalls hergestellt werden, ohne Furane als Zwischenprodukte zu verwenden. Neue Synthesen finden sich beispielsweise in Progress in Heterocyclic Chemistry 14, 139 (2002) oder Progress in Heterocyclic Chemistry 7, 130 (1995).

Als Ester-Schutzgruppe Re können die in "Protective Groups in Organic Synthesis", T.H. Greene, P. G. M. Wuts, Wiley-Interscience, 1999, als Schutzgruppen für Ester verwendete Gruppen eingesetzt werden. Bevorzugte Ester-Schutzgruppen sind beispielsweise Methyl, Ethyl, Isopropyl, tertiär Butyl oder Benzyl.

Unter bestimmten Bedingungen kann es sinnvoll sein, Verbindungen der Formel IV in N-geschütztem Zustand einzusetzen. Beispielsweise können derart geschützte Verbindungen besser aufgereinigt werden als die freien Iminosäuren, ebenso können diese auch besser zur Herstellung der enantiomeren- oder diastereomerenreinen Verbindungen eingesetzt werden. Als Schutzgruppen der Aminogruppe können die in "Protective Groups in Organic Synthesis", T.H. Greene, P. G. M. Wuts, Wiley-Interscience, 1999, beschriebenen Gruppen eingesetzt werden. Bevorzugte Amino- oder Imino-Schutzgruppen sind beispielsweise Z, Boc, Fmoc, Aloc, Acetyl, Trifluoracetyl, Benzoyl, Benzyl und ähnliche Schutzgruppen.

Die eingesetzten Ausgangsprodukte und Reagenzien können entweder nach bekannten Verfahren hergestellt werden oder sind käuflich erhältlich.

Die Umsetzungen erfolgen wie in WO97/18194 dargestellt. Die Umsetzung gemäß Verfahrensschritt a) erfolgt in Gegenwart einer Base wie KOH, NaOH, LiOH, N-Methylmorpholin (NMM), N-Ethylmorpholin (NEM), Triethylamin (TEA), Diisopropylethylamin (DIPEA), Pyridin, Collidin, Imidazol oder Natriumcarbonat, in Lösungsmitteln wie Tetrahydrofuran (THF), Dimethylformamid (DMF), Dimethylacetamid, Dioxan, Acetonitril, Toluol, Chloroform oder Methylenchlorid, oder auch in Gegenwart von Wasser. Für den Fall, dass die Umsetzung unter Verwendung von Silylierungsmitteln durchgeführt wird, setzt man beispielsweise N,O-Bis-(trimethylsilyl)acetamid (BSA) oder N,O-Bis-(trimethylsilyl)trifluoracetamid (BSTFA) zur Silylierung der Iminosäure ein, um anschließend die Sulfonamidbildung wie beschrieben durchzuführen.

Im Verfahrensschritt c) wird die Verbindung der Formel II, sofern sie als Gemisch von Diastereomeren oder Enantiomeren auftritt oder bei der gewählten Synthese als deren Gemische anfällt, in die reinen Stereoisomeren getrennt, entweder durch Chromatographie an einem gegebenenfalls chiralen Trägermaterial, oder, sofern die racemische Verbindung der Formel II zur Salzbildung befähigt ist, durch fraktionierte Kristallisation der mit einer optisch aktiven Base oder Säure als Hilfsstoff gebildeten diastereomeren Salze. Als chirale Stationärphasen für die dünnschicht- oder säulenchromatographische Trennung von Enantiomeren eignen sich zum Beispiel modifizierte Kieselgelträger (sogenannte Pirkle-Phasen) sowie hochmolekulare Kohlenhydrate wie Triacetylcellulose. Für analytische Zwecke sind nach entsprechender, dem Fachmann bekannter Derivatisierung, auch gaschromatographische Methoden an chiralen Stationärphasen anwendbar. Zur Enantiomerentrennung der racemischen Carbonsäuren werden mit einer optisch aktiven, in der Regel kommerziell erhältlichen Base wie (-)-Nicotin, (+)- und (-)-Phenyl-ethylamin, Chininbasen, L-Lysin oder L-und D-Arginin die unterschiedlich löslichen diastereomeren Salze gebildet, die schwerer lösliche Komponente als Feststoff isoliert, das leichter lösliche Diastereomer aus der Mutterlauge abgeschieden und aus den so gewonnenen diastereomeren Salzen die reinen Enantiomeren gewonnen. Auf prinzipiell gleiche Weise kann man die racemischen Verbindungen

der Formel II, die eine basische Gruppe wie eine Aminogruppe enthalten, mit optisch aktiven Säuren, wie (+)-Campher-10-sulfonsäure, D- und L-Weinsäure, D-und L- Milchsäure sowie (+) und (-)-Mandelsäure in die reinen Enantiomeren überführen. Auch kann man chirale Verbindungen, die Alkohol- oder Amin-funktionen enthalten, mit entsprechend aktivierten oder gegebenenfalls N-geschützten enantiomerenreinen Aminosäuren in die entsprechenden Ester oder Amide, oder umgekehrt chirale Carbonsäuren mit carboxygeschützten enantiomerenreinen Aminosäuren in die Amide oder mit enantiomerenreinen Hydroxycarbonsäuren wie Milchsäure, in die entsprechenden chiralen Ester überführen. Sodann kann die Chiralität des in enantiomerenreiner Form eingebrachten Aminosäure- oder Alkoholrestes zur Trennung der Isomeren genutzt werden, indem man eine Trennung der nunmehr vorliegenden Diastereomeren durch Kristallisation oder Chromatographie an geeigneten Stationärphasen vornimmt und danach den mitgeführte chiralen Molekülteil mittels geeigneter Methoden wieder abspaltet.

Weiterhin ergibt sich bei einigen der erfindungsgemäßen Verbindungen die Möglichkeit, zur Herstellung der Gerüststrukturen diastereo- oder enantiomerenrreine Ausgangsprodukte einzusetzen. Dadurch können auch andere oder vereinfachte Verfahren zur Aufreinigung der Endprodukte eingesetzt werden. Diese Ausgangsprodukte wurden zuvor nach Literatur-bekannten Verfahren enantiomeren- oder diastereomerenrein hergestellt. Beispielsweise kann in dem Verfahren zur Herstellung der Octahydro-furo[3,2-c]pyridin-4-carbonsäure entweder direkt die 4,5,6,7-Tetrahydrofuro[3,2-c]pyridin-4-carbonsäure eingesetzt werden, wie oben angeführt und zitiert. Dadurch, dass 3 Stereozentren vorhanden sind, können in diesem Fall maximal 8 Stereoisomere (4 enantiomere Diasteromerenpaare) gebildet werden. Bedingt durch die Art der Herstellung, beispielsweise Hydrierung, sowie die Ringspannung in dem bicyclischen System sind jedoch bestimmte Stereoisomere stark bevorzugt. So sollte es möglich sein, wie in der Literatur beschrieben, durch geeignete Wahl der Hydrierungsbedingungen (Katalysator, Druck, Lösemittel, Temperatur) z. B. eine starke Bevorzugung der Wasserstoff-Anlagerung an den Positionen der Ringverknüpfung zu erzielen. So kann unter den angegebenen Bedingungen die Bildung der cis-verknüpften Ringe erreicht werden. Somit bliebe dann noch die Position der Carbonsäure zu bestimmen, die Anzahl der möglichen Stereoisomeren wäre bereits auf 4 eingeschränkt. Bedingt durch die Natur des Hydriermechanismus kann besonders leicht eine Anlagerung der Wasserstoffe auf der gleichen Seite wie die der Brückenkopf-Wasserstoffe erfolgen, d. h. hiermit ist eine weitere Einschränkung der Möglichkeit der Isomerenbildung zu erwarten. Somit könnte im günstigsten Fall von der Bildung nur eines Enantiomerenpaares ausgegangen werden. Dieses sollte anschließend durch die oben genannten Methoden in die Enantiomeren auftrennbar sein. Allerdings muss bei diesen Überlegungen auch davon ausgegangen werden, dass niemals eine völlige Stereoselektion stattfindet, sondern dass praktisch immer auch zu mehr oder minder großen Anteilen die anderen Isomeren entstehen oder durch geeignete Methoden auch in kleinsten Mengen nachgewiesen werden können.

Für den Fall, dass enantiomerenreine Tetrahydrofuro[3,2-c]pyridin-4-carbonsäureDerivate eingesetzt werden, wäre zu erwarten, dass wieder nur bevorzugte Stereoisomere gebildet werden; in genannten Fall sollte eine starke Bevorzugung eines einzigen Enantiomeren stattfinden, da bei dem Hydrierprozess unter analogen Bedingungen, die zur cis-Ringverknüpfung bei der Hydrierung führen, hier ebenso wieder nur Anlagerung der H-Atome von einer Seite erfolgen kann und somit analoge Produkte gebildet werden. Durch geeignete 2D-NMR-Experimente, Röntgenstrukturanalyse wie etwa der Kristallstrukturanalyse oder Kokristallisation oder andere, sowie Vergleichsanalytik oder chemische Derivatisierung und geeignete Analytik oder chemische Derivatisierung , die zu bekannten und beschriebenen Isomeren führt, kann die Identität der Strukturen festgestellt werden.

Eine andere Möglichkeit zur Synthese enantiomeren- oder diastereomerenreiner Verbindungen besteht darin, geeignet chiral substituierte Ausgangsstoffe einzusetzen, um durch den chiralen Substituenten eine Induktion von Chiralität an anderen Chiralitätszentren zu erreichen. Beispielsweise könnten chirale Glyoxylsäureester in Pictet-Spengler-Cyclisierungen eingesetzt werden, um chirale Furan-Derivate zu erhalten und diese dann, wie oben bereits erwähnt, zu hydrieren.

Saure oder basische Produkte der Verbindung der Formel II können in Form ihrer Salze oder in freier Form vorliegen. Bevorzugt sind pharmakologisch verträgliche Salze, beispielsweise Alkali- oder Erdalkalimetallsalze bzw. Hydrochloride, Hydrobromide, Sulfate, Hemisulfate, alle möglichen Phosphate sowie Salze der Aminosäuren, natürlicher Basen oder Carbonsäuren.

Die Herstellung physiologisch verträglicher Salze aus zur Salzbildung befähigten Verbindungen der Formel II, einschließlich deren stereoisomeren Formen, gemäß Verfahrensschritt d) erfolgt in an sich bekannter Weise. Die Verbindungen der Formel II bilden mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin, Diethanolamin oder Triethanolamin, Trometamol oder auch basischen Aminosäuren, etwa Lysin, Ornithin oder Arginin, stabile Alkali-, Erdalkali- oder gegebenenfalls substituierte Ammoniumsalze. Sofern die Verbindungen der Formel II basische Gruppen aufweisen, lassen sich mit starken Säuren auch stabile Säureadditionssalze herstellen. Hierfür kommen sowohl anorganische als auch organische Säuren wie Chlorwasserstoff-, Bromwasserstoff-, Schwefel-, Hemischwefel-, Phosphor-, Methansulfon-, Benzolsulfon-, p-Toluolsulfon-, 4-Brombenzol-sulfon-, Cyclohexylamidosulfon-, Trifluormethylsulfon-, 2-Hydroxyethansulfon-, Essig-, Oxal-, Wein-, Bernstein-, Glycerolphosphor-, Milch-, Äpfel-, Adipin-, Citronen-, Fumar-, Malein-, Glucon-, Glucuron- Palmitin-, oder Trifluoressigsäure in Frage.

Die Erfindung betrifft auch Arzneimittel, gekennzeichnet durch einen wirksamen Gehalt an mindestens einer Verbindung der Formel II und/oder eines physiologisch verträglichen Salzes der Verbindung der Formel II und/oder eine gegebenenfalls stereoisomere Form der Verbindung der Formel II, zusammen mit einem pharmazeutisch geeigneten und physiologisch verträglichen Trägerstoff, Zusatzstoff und/oder anderen Wirk- und Hilfsstoffen.

Aufgrund der pharmakologischen Eigenschaften eignen sich die erfindungsgemäßen Verbindungen zur selektiven Prophylaxe und Therapie all solcher Erkrankungen, an deren Verlauf eine verstärkte Aktivität der Metalloproteinasen beteiligt sind. Dazu gehören degenerative Gelenkerkrankungen wie Osteoarthrosen, Spondylosen, Knorpelschwund nach Gelenktrauma oder längerer Gelenksruhigstellung nach Meniskus- oder Patellaverletzungen oder Bänderrissen. Ferner gehören dazu auch Erkrankungen des Bindegewebes wie Kollagenosen, Periodontalerkrankungen, Wundheilungsstörungen und chronische Erkrankungen des Bewegungsapparates wie entzündliche, immunologisch oder stoffwechselbedingte akute und chronische Arthritiden, Arthropathien, Myalgien und Störungen des Knochenstoffwechsels. Ferner eignen sich die Verbindungen der Formel II zur Behandlung der Ulceration, Atherosklerose und Stenosen. Weiterhin eignen sich die Verbindungen der Formel II zur Behandlung von Entzündungen, Krebserkrankungen, Tumormetastasenbildung, Kachexie, Anorexie, Herzversagen und septischem Schock. Ebenso eignen sich die Verbindungen zur Prophylaxe von Myocard- und Cerebral-Infarkten.

Die Applikation der erfindungsgemäßen Arzneimittel kann durch orale, inhalative, rektale oder transdermale Applikation oder durch subkutane, intraartikuläre, intraperitoneale oder intravenöse Injektion erfolgen. Bevorzugt ist die orale Applikation.

Die Erfindung betrifft auch ein Verfahren zur Herstellung eines Arzneimittels, das dadurch gekennzeichnet, dass man mindestens eine Verbindung der Formel II mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuß- oder Sesamöl, Polyethylen-glykol und Lösungsmittel wie etwa steriles Wasser und ein- oder mehrwertige Alkohole wie Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel II enthält. Bei festen Dosierungseinheiten wie Tabletten, Kapseln, Dragees oder Suppositorien, kann diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen.

Für die Behandlung eines erwachsenen, etwa 70 kg schweren Patienten sind je nach Wirksamkeit der Verbindung gemäß Formel II, Tagesdosen von etwa 2 mg bis 1000 mg Wirkstoff, bevorzugt etwa 50 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Endprodukte werden in der Regel durch massenspektroskopische Methoden (FAB-, ESI-MS) und $^{1}$H-NMR (500 MHz, in DMSO-D6)bestimmt, angegeben sind jeweils der Hauptpeak oder die beiden Hauptpeaks. Temperaturangaben in Grad Celsius, RT bedeutet Raumtemperatur (21 °C bis 24 °C). Verwendete Abkürzungen sind entweder erläutert oder entsprechen den üblichen Konventionen.

Nachfolgend ist die Erfindung an Hand von Beispielen näher erläutert.

Allgemeine Vorschrift 1: Sulfonamid aus Sulfonsäurechlorid und Carbonsäure

Die Carbonsäure (6,45 mmol) wurde in 20 ml Dimethylformamid (DMF) gelöst und bei 0 °C mit 3 Äquivalenten einer 3N NaOH-Lösung (6,45 ml) versetzt. Nach 10 min tropfte man eine Lösung des Arylsulfonylchlorids (1,1 Äquivalente, 7,1 mmol) in 10 bis 15 ml DMF langsam zu, nach dem Erreichen der Raumtemperatur (RT) wird der Ansatz noch für maximal 12 Stunden (h) bei Temperaturen zwischen 20 °C und 80 °C gerührt. Die genaue Zeit ergibt sich je nach erfolgtem Umsatz, der massenspektroskopisch festgestellt wurde. Danach wurde das Lösungsmittel unter verminderten Druck entfernt. Anschließend erfolgte wässrige Aufarbeitung (Ausschütteln mit 1 N HCl und gesättigter NaCl-Lösung, Trocknen der organischen Phase wie Essigester, Methylenchlorid oder Chloroform mit Magnesium- oder Natriumsulfat, danach Einengen). Das Rohprodukt wurde entweder direkt weiter umgesetzt oder chromatographisch gereinigt.

Allgemeine Vorschrift 2: Sulfonamid aus Sulfonsäurechlorid und Carbonsäure

Die Carbonsäure wurde in 0,5-2 molarer NaOH gelöst, eventuell unter Zugabe von 10-50 % Tetrahydrofuran (THF) oder DMF. Säurechlorid (1-1,2 Äquivalente, bevorzugt 1,1) wurde in THF gelöst (Konzentration 0,05 bis 1 M) und langsam zugetropft. Am Autotitrator erfolgte automatisch Zugabe von 2 N NaOH bei RT zur pH-Konstanthaltung. Eingestellter pH-Wert: 8 bis 12, bevorzugt 9 bis 11. Nach Beendigung der Reaktion, erkennbar an keinem weiteren NaOH-Verbrauch, wurde das organische Co-Lösungsmittel am Rotationsverdampfer entfernt, die wässrige Lösung oder Suspension mit Essigester versetzt und mit 1 N HCl angesäuert. Nach Abtrennung der organischen Phase und erneuter Extraktion der wässrigen Phase mit Essigester wurden die organischen Phasen vereinigt, über Natriumsulfat getrocknet und anschließend das Lösemittel unter verminderten Druck entfernt. Das Rohprodukt wurde entweder direkt weiter umgesetzt oder chromatographisch gereinigt.

Allgemeine Vorschrift 3: Sulfonamid aus Sulfonsäurechlorid und Carbonsäure. Diese Vorschrift ist besonders geeignet zur Umsetzung von Biphenylethylsulfonsäurechlorid mit Iminocarbonsäuren (siehe Beispiel 6 und Beispiel 7) oder ähnlichen, hydrolyselabileren Sulfonsäurechloriden.

8 mmol der Iminosäure wurden in 30 ml Acetonitril gelöst oder suspendiert. Bei RT und unter Inertgas ($N_2$) wurden 2,3 g (9 mmol) BSTFA (Bis-(trimethylsilyl)-trifluoracetamid) zugegeben und die Mischung für 2 h unter Rückfluss erhitzt. Zu dieser Lösung gab man 2,84 g (9 mmol) 4-Chlorbiphenylethansulfonylchlorid, gelöst in 30 ml Acetonitril und erhitzte unter Rückflussbedingungen erneut für 3 h. Nach Abkühlen der Reaktionsmischung gab man wässrige 1 N HCl zu, rührte für 1 h, entfernte das Lösemittel unter verminderten Druck am Rotationsverdampfer und gab anschließend Essigsäurethylester oder Chloroform hinzu, trennte die organische Phase ab, extrahierte diese mit gesättigter NaCl-Lösung , trocknete über Natriumsulfat und engte unter verminderten Druck ein. Je nach Reinheit des Reaktionsproduktes konnte dieses direkt weiter umgesetzt werden oder musste vorher über Kieselgel chromatographiert werden.

Allgemeine Vorschrift 4: Herstellung der Hydroxamsäure aus Carbonsäure über Chloroformat-Aktivierung

Die sulfonierte Carbonsäure wurde in 10 ml DMF gelöst und bei 0 °C mit 1,1 Äquivalenten Ethylchloroformiat, 2,2 Äquivalenten N-Ethylmorpholin sowie - nach einer Voraktivierungszeit von 30 min bis 1 h - mit 3 Äquivalenten Trimethylsilylhydroxylamin versetzt. Nachdem man für mindestens 4 h auf 80 °C erhitzte hat, entfernte man das Lösungsmittel unter verminderten Druck und reinigte das Rohprodukt mit chromatographischen Methoden.

Allgemeine Vorschrift 5: Herstellung der Hydroxamsäure durch über das korrespondierende Carbonsäurechlorid

Die sulfonierte Carbonsäure wurde in trockenem Chloroform (Ethanol-frei) vorgelegt (etwa 5 ml für 0,5 mmol) und bei RT mit 3 Äquivalenten Oxalylchlorid versetzt. Anschließend wurde für etwa 30 min auf 45 °C erwärmt. Zur Kontrolle der Chloridbildung wurde eine kleine Probe aus dem Reaktionskolben herausgenommen und mit wenig Benzylamin in THF versetzt. Die vollständige Umsetzung konnte an der quantitativen Benzylamid-Bildung erkannt werden, die Carbonsäure war nicht mehr nachweisbar (Kontrolle durch HPLC-MS). Gegebenenfalls muss für längere Zeit erwärmt werden oder unter Rückflussbedingungen erhitzt werden. Anschließend wurde das Lösemittel unter verminderten Druck abdestilliert, der Rückstand wurde mehrfach in trockenem Toluol aufgenommen und erneut einrotiert. Das Säurechlorid wurde nun erneut in Chloroform (10 ml pro 0,5 mmol) aufgenommen und bei RT mit 3 Äquivalenten O-Trimethylsilylhydroxylamin versetzt. Nach einer Reaktionszeit von mindestens 30 min (Reaktionskontrolle per HPLC-MS) wurde die Reaktionsmischung unter verminderten Druck eingedampft und der Rückstand direkt chromatographisch gereinigt.

Spezielle Vorschriften

Beispiel 1: Octahydro-furo[3,2-c]pyridin-4-carbonsäure

Man löste 2,5 g des entsprechenden Furan-Derivates 4,5,6,7-Tetrahydro-furo[3,2-c]pyridin-4-carbonsäure (167,16; 14,96 mmol)) in Methanol (MeOH; 45 ml) und hydrierte bei 5 bar und RT mit 0,5 g Rhodium auf Aluminiumoxid für 38 h. Nach Reaktionskontrolle wurde anschließend vom Katalysator abfiltriert, mit Acetonitril gewaschen und die verbliebene gelbliche Lösung wurde nach Zugabe von 15 ml 1 M HCl unter verminderten Druck eingeengt. Der wässrige Rückstand wurde eingefroren und gefriergetrocknet.
Ausbeute: 1,51 g (53 % der Theorie)

Beispiel 2: N-(4-Chlorbiphenylsulfonyl)-4,5,6,7-tetrahydro-furo[3,2-c]pyridin-4-carbonsäure

Man löste oder suspendierte die nach oben angegebener Literaturvorschrift hergestellte Iminosäure 4,5,6,7-Tetrahydro-furo[3,2-c]pyridin-4-carbonsäure (250 mg, 1,5 mmol) in Acetonitril (15 ml) und erhitzte zusammen mit N,0-Bis-(trimethylsilyl)-acetamid (671 mg, 0,82 ml, 3,3 mmol) für 45 min unter Ruckfluss. Anschließend wurde 4-Chlorbiphenyl-sulfonylchlorid (473,8 ml, 1,65 mmol, 1,1 eq.), gelöst in 5 ml Acetonitril, nach Abkühlen zugegeben. Nach einer weiteren Stunde am Rückfluss wurde die Reaktionsmischung unter verminderten Druck eingeengt, in Essigsäureethylester aufgenommen und mit verdünnter Salzsäure oder gesättigter Natriumchlorid-Lösung ausgeschüttelt. Die vereinigten organischen Phasen wurden über Natriumsulfat getrocknet. Nach Entfernen des Lösemittels verblieb ein öliger Rückstand, der im Ölpumpenvakuum fest wurde und für weitere Umsetzungen hinreichend rein war.
Ausbeute: 455 mg g (73 % der Theorie). Analytische Daten: siehe Tabelle 1.

Beispiel 3: N-(4-Chlorbiphenylsulfonyl)- 4,5,6,7-tetrahydro-furo[3,2-c]pyridin-4-N-hydroxycarboxamid

Die Carbonsäure aus Beispiel 2 (430 mg, 1,03 mmol) wurde in 20 ml Chloroform gelöst. Anschließend wurde Oxalylchlorid (2,176 g, 17,14 mmol, 1,501 ml) innerhalb von 10 min zugetropft und die erhaltene Reaktionsmischung für eine Stunde auf 45 °C erwärmt. Nach dieser Zeit wurde zur Reaktionskontrolle per HPLC-MS eine kleine Probe der Reaktionsmischung (0,1 ml) entnommen und mit 0,05 ml Benzylamin versetzt. Anschließend wurde das Lösemittel unter verminderten Druck abdestilliert und der erhaltene ölige Rückstand mit Toluol zur Entfernung von etwaigen Oxalylchlorid-Resten oder HCl geschleppt und unter verminderten Druck 15 min belassen. Dann wurde wiederum in Chloroform (15 ml) aufgenommen und bei RT mit O-Trimethylsilylhydroxylamin (325,1 mg, 3,09 mmol, 0,378 ml) versetzt. Nach 2 Stunden wurde das Lösemittel unter verminderten Druck entfernt und der Rückstand in einer kleinen Menge einer Mischung aus Acetonitril-Wasser-0,01 % Trifluoressigsäure zur direkten präparativen RP-HPLC gelöst. Produktfraktionen wurden vereinigt, Acetonitril unter verminderten Druck entfernt und die verbleibende wässrige Phase wurde gefriergetrocknet. Ausbeute: 20 mg (7 % der Theorie, daneben erhält man 110 mg einer verunreinigten Fraktion Analytische Daten: siehe Tabelle 1.

Die nachfolgenden Beispiele wurden analog zu den vorher genannten allgemeinen oder speziellen Vorschriften hergestellt. Tabelle 1 zeigt die Ergebnisse.

Tabelle 1

| Beispiel | Struktur | Molgewicht | $ES^+$ | 1H-NMR |
|---|---|---|---|---|
| 1 | 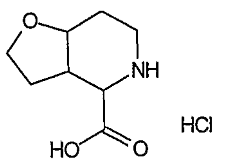 | 171,10 | 172,20 | 1.4-2.05 (3 m, 3 H); 2.6-4.1 (mm, 8 H, das NMR-Signal von $H_2O$ überlappt mit dem Signal der Substanz) |
| 2 | 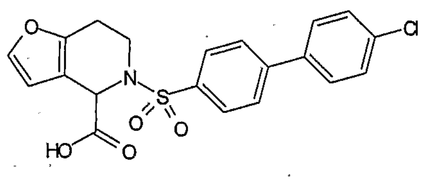 | 417,87 | 418,03 | 2.55; 3.55; 4.10 (3 m, 4 H); 5.39; 6.45 (2 s, 2 H); 7.53; 7.82 (2 m, 9 h); 13.2 (s, 1 H) |
| 3 | 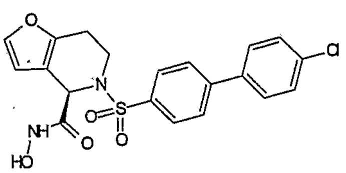 | 432,89 | 433,06 | 2.55; 3.85; 4.10 (3 m, 4 H); 5.20; 6.35 (2 s, 2 H); 7.50; 7.82 (2 m, 9 h); 11.0 (s, 1 H) |

(fortgesetzt)

| Beispiel | Struktur | Molgewicht | ES+ | 1H-NMR |
|---|---|---|---|---|
| 4 | 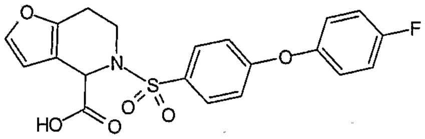 | 417,41 | 418,04 | 2.55; 3.55; 4.10 (3 m, 4 H, das NMR-Signal von $H_2O$ überlappt mit dem Signal der Substanz); 5.42; 6.45 (2 s, 2 H); 7.0; 7.18 (2 m, 4 H); 7.30 (m, 2 H); 7.51 (s, 1 H); 7.8 (m, 2 H); 13.2 (s, 1 H) |
| 5 | 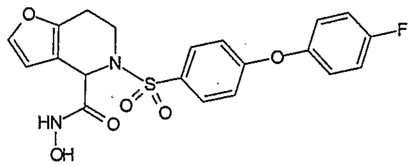 | 432,43 | 433,07 | 2.55; 3.8; 4.0 (3 m, 4 H, das NMR-Signal von $H_2O$ überlappt mit dem Signal der Substanz); 5.12; 6.33 (2 s, 2 H); 7.0; 7.18 (2 m, 4 H); 7.30 (m, 2 H); 7.51 (s, 1 H); 7.74 (m, 2 H); 11.2 (s, 1 H) |
| 6 | 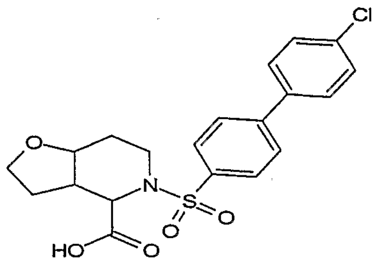 | 421,90 | 422,25 | 1.5-2.15 (4 m, 3-4 H); 2.55 (m, 1 H); 3.25-3.8 (4m, 4-5 H, das NMR-Signal von $H_2O$ überlappt mit dem Signal der Substanz); 4.33 (d, 1 H); 7.6, 7.8 (dd, 4 H); 7.9 (dd, br, 4 H); 12.9 (s, 1 H) |
| 7 | 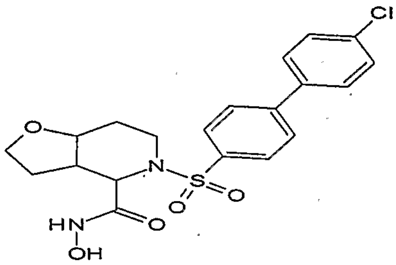 | 436,92 | 437,28 | 1.5-2.0 (3 m, 3-4 H); 2.4 (m, 1 H); 3.2-3.8 (5m, 4-5 H, das NMR-Signal von $H_2O$ überlappt mit dem Signal der Substanz); 4.2 (d, 1 H); 7.1, 7.8 (dd, 4 H); 7.2, 7.3 (dd, br, 4 H); 10.8 (s, 1 H) |
| 8 | CHIRAL 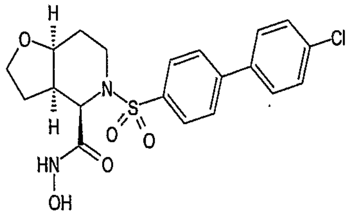 | 438,93 | | aus chiraler Auftrennung von Beispiel 7 |
| 9 | CHIRAL 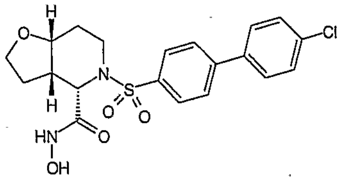 | 438,93 | | aus chiraler Auftrennung von Beispiel 7 |

(fortgesetzt)

| Beispiel | Struktur | Molgewicht | ES+ | 1H-NMR |
|---|---|---|---|---|
| 10 | 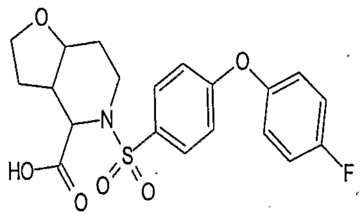 | 421,45 | 422,26 | 1.5-2.1 (4 m, 4 H); 2.55 (m, 1 H); 3.25-3.85 (4m, 4-5 H, das NMR-Signal von $H_2O$ überlappt mit dem Signal der Substanz); 4.28 (d, 1 H); 7.1 (d, 2 H); 7.2-7.35 (2m, 4 H); 7.8 (d, br, 2 H); 12.9 (s, 1 H) |
| 11 | 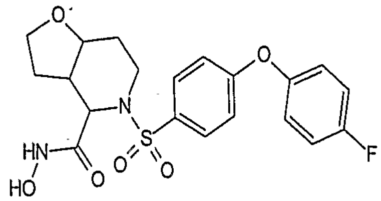 | 436,46 | 437,25 | 1.5-2.0 (3 m, 4 H); 2.4 (m, 1 H); 3.4-3.85 (4m, 4-5 H, das NMR-Signal von $H_2O$ überlappt mit dem Signal der Substanz); 4.23 (d, 1 H); 7.1 (d, 2 H); 7.6-7.95 (4"d", 8 H); 10.8 (s, 1 H) |
| 12 | 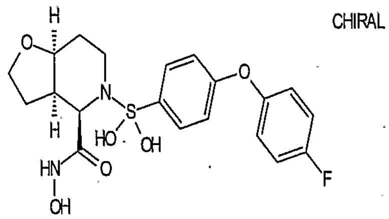 | 438,48 | | aus chiraler Auftrennung von Beispiel 11 |
| 13 | 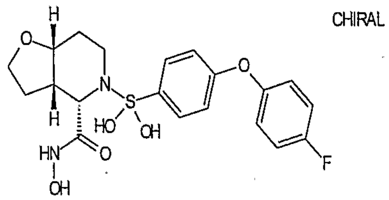 | 438,48 | | aus chiraler Auftrennung von Beispiel 11 |
| 14 | 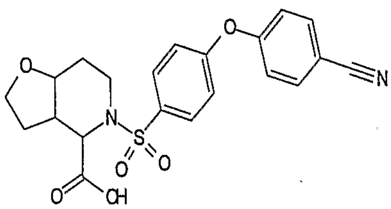 | 428,47 | 428,10 | 1.5-2.15 (4 m, 4 H); 2.5 (m, 1 H); 3.25-3.80 (4 m, 4-5 H, das NMR-Signal von $H_2O$ überlappt mit dem Signal der Substanz); 4.05 (m, 1 H); 4.3 (d, 1 H); 7.0-7.5 (4 m, 4 H); 7.9 (m, 4 H); 13.0 (s, br, 1 H) |
| 15 | 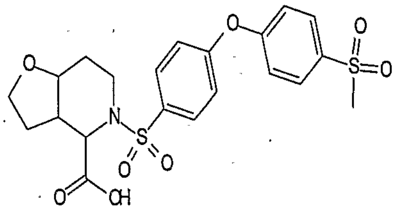 | 481,55 | 481,09 | 1.55-2.1 (4 m, 4 H); 2.5 (m, 1 H); 3.25-3.80 (4 m, 4-5 H, das NMR-Signal von $H_2O$ überlappt mit dem Signal der Substanz); 4.0 (m, 1 H); 4.3 (d, 1 H); 7.3 (m, 4 H); 7.85; 8.0 (2 m, 4 H); 13.0 (s, br, 1 H) |

(fortgesetzt)

| Beispiel | Struktur | Molgewicht | ES+ | 1H-NMR |
|---|---|---|---|---|
| 16 | 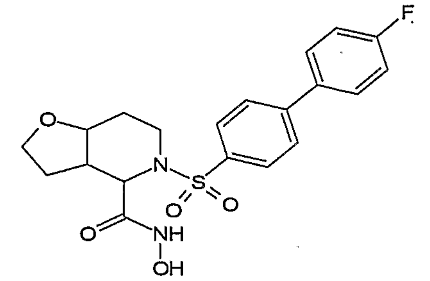 | 420,46 | 421,10 | 1.5-2.0 (3 m, 4 H); 2.4 (m, 1 H); 3.25-3.8 (3 m, 4-5 H, das NMR-Signal von $H_2O$ überlappt mit dem Signal der Substanz); 4.22 (d, 1 H); 7.37 (m, 2 H); 7.85 (m, 6 H); 8.9 (s, 1 H); 10.8 (s, 1H) |
| 17 | 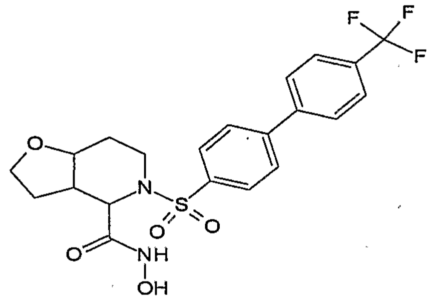 | 470,47 | 471,11 | 1.5-2.0 (3 m, 4 H); 2.4 (m, 1 H); 3.25-3.8 (4 m, 4-5 H, das NMR-Signal von $H_2O$ überlappt mit dem Signal der Substanz); 4.26 (d, 1 H); 7.9 (m, 8 H); 8.9 (s, 1 H); 10.8 (s. 1 H) |
| 18 | 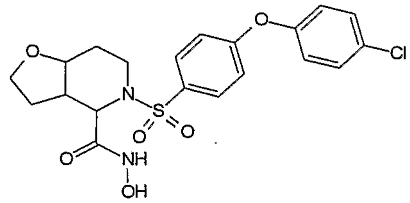 | 452,92 | 452,97 | 1.5-2.0 (3 m, 4 H); 2.4 (m, 1 H); 3.4-3.85 (4m, 4-5 H, das NMR-Signal von $H_2O$ überlappt mit dem Signal der Substanz); 4.2 (d, 1 H); 7.2 (2 d, 4 H); 7.5; 7.8 (2 d, 4H); 10.8 (s, 1 H) |
| 19 | 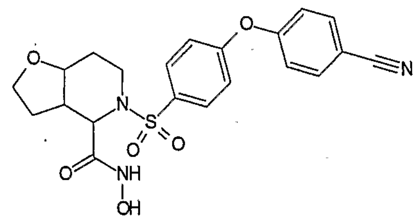 | 443,48 | 444,12 | 1.5-2.0 (3 m, 4 H); 2.4 (m, 1 H); 3.2-3.8 (5m, 5 H, das NMR-Signal von $H_2O$ überlappt mit dem Signal der Substanz); 4.2 (d, 1 H); 7.3 (m, 4 H); 7.8; 7.9 (2 d, 4 H); 8.9 (s, 1 H); 10.8 (s, 1 H) |
| 20 | 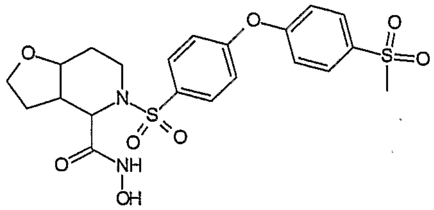 | 496,56 | 497,11 | 1.5-2.0 (4 m, 4 H); 2.4 (m, 1 H); 3.2-3.8 (5 m, 5 H, das NMR-Signal von $H_2O$ überlappt mit dem Signal der Substanz); 4.2 (d, 1 H); 7.3 (2 d, 4 H); 7.85; 8.0 (2 d, 4H); 8.9 (s, 1 H); 10.8 (s, 1 H) |

(fortgesetzt)

| Beispiel | Struktur | Molgewicht | ES+ | 1H-NMR |
|---|---|---|---|---|
| 21 | 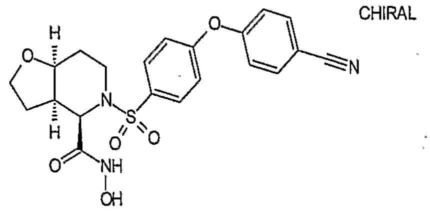 | 443,48 | | aus chiraler Auftrennung example 19 |
| 22 | 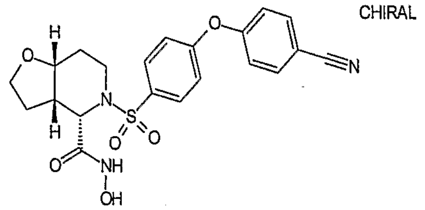 | 443,48 | | aus chiraler Auftrennung von Beispiel 19 |
| 23 | 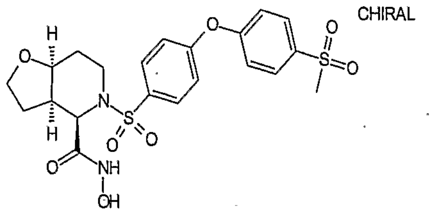 | 496,56 | | aus chiraler Auftrennung von Beispiel 20 |
| 24 | 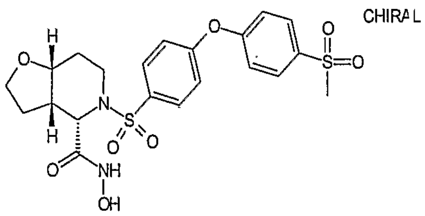 | 496,56 | | aus chiraler Auftrennung von Beispiel 20 |

Pharmakologische Beispiele

Bestimmung der enzymatischen Aktivität der katalytischen Domäne der humanen Kollagenase-1 (MMP-1).

Dieses Protein wird als inaktives Pro-Enzym von der Fa. Biocol, Potsdam, erhalten (Katalog Nr. MMP1). Aktivierung des Proenzyms:

2 Volumenanteile Proenzym werden mit 1 Volumenanteil APMA-Lösung bei 37 °C für 1 Stunde inkubiert. Die APMA-Lösung wird aus einer 10 mmol/L p-Aminophenyl-Mercuric Acetate Lösung in 0,1 mmol/L NaOH durch Verdünnen mit 3 Volumenteile Tris/HCl Puffer pH7,5 (siehe unten) hergestellt. Der pH-Wert wird durch Zugabe von 1 mmol/L HCl zwischen 7,0 und 7,5 eingestellt. Nach der Aktivierung des Enzyms wird dieses mit dem Tris/HCl Puffer auf eine Konzentration von 2,5 µg/mL verdünnt.

Zur Messung der Enzymaktivität werden 10 µL Enzymlösung mit 10 µL einer 3%igen (v/v) gepufferten Dimethylsulfoxid-Lösung (Reaktion 1) für 15 Minuten inkubiert. Zur Messung der Enzyminhibitoraktivität werden 10 µL Enzymlösung mit 10 µL einer 3%igen (v/v) gepufferten Dimethylsulfoxid-Lösung, die den Enzyminhibitor enthält, inkubiert (Reaktion 2).

Sowohl bei Reaktion 1 als auch bei Reaktion 2 wird nach Zugabe von 10 µL einer 3%igen (v/v) wässrigen Dimethylsulfoxid-Lösung, die 0,3 mmol/L des Substrates enthält, die Enzymreaktion fluoreszenzspektroskopisch verfolgt (328 nm (Extinktion) / 393 nm(Emission)).

Die Enzymaktivität wird dargestellt als Extinktionszunahme/Minute.

Die Inhibitorwirkung wird als prozentuale Hemmung nach folgender Formel berechnet:

$$\% \text{ Hemmung} = 100 - [(\text{Extinktionszunahme/Minute in Reaktion 2}) / (\text{Extinktionszunahme/Minute in Reaktion 1}) \times 100].$$

Der $IC_{50}$, d.h. die für eine 50%ige Hemmung der Enzymaktivität erforderliche Inhibitorkonzentration wird grafisch durch Auftragen der prozentualen Hemmungen bei verschiedenen Inhibitorkonzentrationen ermittelt.
Die Pufferlösung enthält 0,05% Brij (Sigma, Deisenhofen, Deutschland) sowie
0,1 mol/L Tris/HCl, 0,1 mol/L NaCl, 0,01 mol/L $CaCl_2$ (pH=7,5).
Die Enzymlösung enthält 2,5 μg/mL der Enzymdomäne.
Die Substratlösung enthält 0,3 mmol/L des fluorogenen Substrates (7-Methoxycoumarin-4-yl)acetyl-Pro-Leu-Gly-Leu-3-(2',4'-dinitrophenyl)-L-2,3-diaminopropionyl-Ala-Arg-$NH_2$ (Bachem, Heidelberg, Deutschland).
Darstellung und Bestimmung der enzymatischen Aktivität der katalytischen Domäne des humanen Stromelysins (MMP-3) und der Neutrophilen-Kollagenase (MMP-8).
Die beiden Enzyme -Stromelysin (MMP-3) und Neutrophilen-Kollagenase (MMP-8) - wurden dargestellt nach Ye et al. (Biochemistry; 31 (1992) Seiten 11231-11235). Zur Messung der Enzymaktivität oder der Enzyminhibitorwirkung wurden 10 μl Enzymlösung mit 10 μl einer 3 %igen (v/v) gepufferten Dimethylsulfoxid-Lösung, die gegebenenfalls den Enzyminhibitor enthielt, für 15 Minuten inkubiert. Nach Zugabe von 10 μl einer 3 %igen (v/v) wässrigen Dimethylsulfoxid-Lösung, die 1 mmol/l des Substrates enthielt, wurde die Enzymreaktion fluoreszenzspektroskopisch verfolgt (328 nm (ex) / 393 nm(em)).
Die Enzymaktivität wird dargestellt als Extinktionszunahme/Minute. Die in Tabelle 2 aufgeführten $IC_{50}$-Werte wurden als diejenige Inhibitorkonzentrationen ermittelt, die jeweils zu einer 50%igen Inhibierung des Enzyms führte.
Die Pufferlösung enthielt 0,05 % Brij (Sigma, Deisenhofen, Deutschland) sowie 0,1 mol/l Tris/HCl, 0,1 mol/l NaCl, 0,01 mol/l $CaCl_2$ und 0,1 mol/l Piperazin-N,N'-bis[2-ethan-sulfonsäure] (pH=7,5).
Die MMP-3 Enzymlösung enthielt 2,3 μg/ml, die MMP-8 Enzymlösung 0,6 μg/ml einer der nach Ye et al. dargestellten Enzymdomänen. Die Substratlösung enthielt 1 mmol/l des fluorogenen ) Substrates (7-Methoxycoumarin-4-yl)acetyl-Pro-Leu-Gly-Leu-3-(2',4'-dinitrophenyl)-L-2,3-diaminopropionyl-Ala-Arg-$NH_2$ (Bachem, Heidelberg, Deutschland).
Bestimmung der enzymatischen Aktivität der katalytischen Domäne der humanen Kollagenase -3 (MMP-13).
Dieses Protein wurde als inaktives Pro-Enzym von der Fa. INVITEK, Berlin, erhalten (Katalog Nr. 30100 803). Aktivierung des Proenzyms:

> 2 Volumenanteile Proenzym wurden mit 1 Volumenanteil APMA-Lösung bei 37 °C für 1,5 Stunden inkubiert. Die APMA-Lösung wurde aus einer 10 mmol/L p-Aminophenyl-Mercuric Acetate Lösung in 0,1 mmol/L NaOH durch Verdünnen mit 3 Volumenteile Tris/HCl Puffer pH7,5 (siehe unten) hergestellt. Der pH-Wert wurde durch Zugabe von 1 mmol/L HCl zwischen 7,0 und 7,5 eingestellt. Nach der Aktivierung des Enzyms wurde dieses mit dem Tris/HCl Puffer auf eine Konzentration von 1,67 μg/mL verdünnt.

Zur Messung der Enzymaktivität wurden 10 μL Enzymlösung mit 10 μL einer 3 %igen (v/v) gepufferten Dimethylsulfoxid-Lösung (Reaktion 1) für 15 Minuten inkubiert. Zur Messung der Enzyminhibitoraktivität wurden 10 μL Enzymlösung mit 10 μL einer 3 %igen (v/v) gepufferten Dimethylsulfoxid-Lösung, die den Enzyminhibitor enthielt, inkubiert (Reaktion 2).
Sowohl bei Reaktion 1 als auch bei Reaktion 2 wurde nach Zugabe von 10 μL einer 3 %igen (v/v) wässrigen Dimethylsulfoxid-Lösung, die 0,075 mmol/L des Substrates enthielt, die Enzymreaktion fluoreszenzspektroskopisch verfolgt (328 nm (Extinktion) / 393 nm(Emission)). Die Enzymaktivität wurde dargestellt als Extinktionszunahme/Minute.
Die Inhibitorwirkung wurde als prozentuale Hemmung nach folgender Formel berechnet:

$$\% \text{ Hemmung} = 100 - [(\text{Extinktionszunahme/Minute in Reaktion 2}) / (\text{Extinktionszunahme/Minute in Reaktion 1}) \times 100].$$

Der $IC_{50}$, dies ist die Inhibitorkonzentration, die für eine 50 %ige Hemmung der Enzymaktivität erforderliche ist, wurde grafisch durch Auftragen der prozentualen Hemmungen bei verschiedenen Inhibitorkonzentrationen ermittelt.
Die Pufferlösung enthielt 0,05% Brij (Sigma, Deisenhofen, Deutschland) sowie 0,1 mol/L Tris/HCl, 0,1 mol/L NaCl, 0,01 mol/L $CaCl_2$ (pH=7,5). Die Enzymlösung enthielt 1,67 μg/mL der Enzymdomäne. Die Substratlösung enthielt 0,075 mmol/L des fluorogenen Substrates (7-Methoxycoumarin-4-yl)acetyl-Pro-Leu-Gly-Leu-3-(2',4'-dinitrophenyl)-L-2,3-diaminopropionyl-Ala-Arg-$NH_2$ (Bachem, Heidelberg, Deutschland).

Bestimmung der enzymatischen Aktivität der katalytischen Domäne der humanen Gelatinase - A (MMP-2).
Dieses Protein wurde als inaktives Pro-Enzym von der Fa. INVITEK, Berlin, erhalten (Katalog Nr. 30 100 602). Aktivierung des Proenzyms:

2 Volumenanteile Proenzym wurden mit 1 Volumenanteil APMA-Lösung bei 37 °C für 0,5 Stunden inkubiert. Die APMA-Lösung wurde aus einer 10 mmol/L p-Aminophenyl-Mercuric Acetate Lösung in 0,1 mmol/L NaOH durch Verdünnen mit 3 Volumenteile Tris/HCl Puffer pH7,5 (siehe unten) hergestellt. Der pH-Wert wurde durch Zugabe von 1 mmol/L HCl zwischen 7,0 und 7,5 eingestellt. Nach der Aktivierung des Enzyms wurde dieses mit dem Tris/HCl Puffer auf eine Konzentration von 0,83 µg/mL verdünnt.

Zur Messung der Enzymaktivität wurden 10 µL Enzymlösung mit 10 µL einer 3 %igen (v/v) gepufferten Dimethylsulfoxid-Lösung (Reaktion 1) für 15 Minuten inkubiert. Zur Messung der Enzyminhibitoraktivität wurden 10 µL Enzymlösung mit 10 µL einer 3 %igen (v/v) gepufferten Dimethylsulfoxid-Lösung, die den Enzyminhibitor enthielt, inkubiert (Reaktion 2).
Sowohl bei Reaktion 1 als auch bei Reaktion 2 wurde nach Zugabe von 10 µL einer 3 %igen (v/v) wässrigen Dimethylsulfoxid-Lösung, die 0,3 mmol/L des Substrates enthielt, die Enzymreaktion fluoreszenzspektroskopisch verfolgt (328 nm (Extinktion) / 393 nm(Emission)). Die Enzymaktivität wurde als Extinktionszunahme/Minute dargestellt.

Die Inhibitorwirkung wurde als prozentuale Hemmung nach folgender Formel berechnet:

% Hemmung = 100 – [(Extinktionszunahme/Minute in Reaktion 2) / (Extinktionszunahme/Minute in Reaktion 1) x 100].

Der $IC_{50}$, dies ist die Inhibitorkonzentration, die für eine 50 %ige Hemmung der Enzymaktivität erforderliche ist, wurde grafisch durch Auftragen der prozentualen Hemmungen bei verschiedenen Inhibitorkonzentrationen ermittelt.
Die Pufferlösung enthielt 0,05% Brij (Sigma, Deisenhofen, Deutschland) sowie 0,1 mol/L Tris/HCl, 0,1 mol/L NaCl, 0,01 mol/L $CaCl_2$ (pH=7,5). Die Enzymlösung enthielt 0,83 µg/mL der Enzymdomäne. Die Substratlösung enthielt 0,3 mmol/L des fluorogenen Substrates (7-Methoxycoumarin-4-yl)acetyl-Pro-Leu-Gly-Leu-3-(2',4'-dinitrophenyl)-L-2,3-diaminopropionyl-Ala-Arg-$NH_2$ (Bachem, Heidelberg, Deutschland).
Bestimmung der enzymatischen Aktivität der katalytischen Domäne der humanen Gelatinase-B (MMP-9).
Dieses Protein wurde als inaktives Pro-Enzym von der Fa. Roche, Mannheim, erhalten (Katalog Nr. 1 758 896). Aktivierung des Proenzyms:

2 Volumenanteile Proenzym wurden mit 1 Volumenanteil APMA-Lösung bei 37 °C für 4 Stunden inkubiert. Die APMA-Lösung wurde aus einer 10 mmol/L p-Aminophenyl-Mercuric Acetate Lösung in 0,1 mmol/L NaOH durch Verdünnen mit 3 Volumenteile Tris/HCl Puffer pH7,5 (siehe unten) hergestellt. Der pH-Wert wurde durch Zugabe von 1 mmol/L HCl zwischen 7,0 und 7,5 eingestellt. Nach der Aktivierung des Enzyms wurde dieses mit dem Tris/HCl Puffer auf eine Konzentration von 4,2 mU/mL verdünnt.

Zur Messung der Enzymaktivität wurden 10 µL Enzymlösung mit 10 µL einer 3 %igen (v/v) gepufferten Dimethylsulfoxid-Lösung (Reaktion 1) für 15 Minuten inkubiert. Zur Messung der Enzyminhibitoraktivität wurden 10 µL Enzymlösung mit 10 µL einer 3 %igen (v/v) gepufferten Dimethylsulfoxid-Lösung, die den Enzyminhibitor enthielt, inkubiert (Reaktion 2).
Sowohl bei Reaktion 1 als auch bei Reaktion 2 wurde nach Zugabe von 10 µL einer 3 %igen (v/v) wässrigen Dimethylsulfoxid-Lösung, die 0,15 mmol/L des Substrates enthielt, die Enzymreaktion fluoreszenzspektroskopisch verfolgt (328 nm (Extinktion) / 393 nm(Emission)). Die Enzymaktivität wurde dargestellt als Extinktionszunahme/Minute.
Die Inhibitorwirkung wurde als prozentuale Hemmung nach folgender Formel berechnet:

% Hemmung = 100 – [(Extinktionszunahme/Minute in Reaktion 2) / (Extinktionszunahme/Minute in Reaktion 1) x 100].

Der $IC_{50}$, dies ist die Inhibitorkonzentration, die für eine 50 %ige Hemmung der Enzymaktivität erforderliche ist, wurde grafisch durch Auftragen der prozentualen Hemmungen bei verschiedenen Inhibitorkonzentrationen ermittelt.
Die Pufferlösung enthielt 0,05% Brij (Sigma, Deisenhofen, Deutschland) sowie 0,1 mol/L Tris/HCl, 0,1 mol/L NaCl, 0,01 mol/L $CaCl_2$ (pH=7,5). Die Enzymlösung enthielt 4,2 mU/mL der Enzymdomäne. Die Substratlösung enthielt

0,15 mmol/L des fluorogenen Substrates (7-Methoxycoumarin-4-yl)acetyl-Pro-Leu-Gly-Leu-3-(2',4'-dinitrophenyl)-L-2,3-diaminopropionyl-Ala-Arg-$NH_2$ (Bachem, Heidelberg, Deutschland).

Die nachfolgende Tabelle 2 zeigt die Ergebnisse.

Tabelle 2:

| Beispiel Nr. | $IC_{50}$ [nM] MMP-1 | $IC_{50}$ [nM] MMP-2 | $IC_{50}$ [nM] MMP-3 | $IC_{50}$ [nM] MMP-8 | $IC_{50}$ [nM] MMP-9 | $IC_{50}$ [nM] MMP-13 |
|---|---|---|---|---|---|---|
| **6** | >10000 | 38 | 3500 | 54 | 2200 | 290 |
| **7** | 29 | 1,7 | 29 | 2,4 | 2,4 | 1,8 |
| **8** | 14 | 0,8 | 13 | 1 | 1,5 | 1 |
| **9** | 4100 | 71 | 1100 | 170 | 120 | 58 |
| **10** | >10000 | 440 | >10000 | 410 | 2500 | 520 |
| **11** | 39 | 2 | 34 | 6 | 3 | 2 |
| **12** | 10 | 0,8 | 14 | 2 | 1,3 | 0,7 |
| **13** | 1400 | 47 | 1500 | 150 | 150 | 63 |
| **18** | 43 | 2 | 27 | 24 | 2 | 1 |
| **21** | 59 | 1 | 17 | 2 | 8 | 1 |
| **23** | 640 | 1 | 15 | 2 | 5 | 1 |
| > bedeutet größer als. | | | | | | |

## Patentansprüche

1. Verbindung der Formel II, wobei

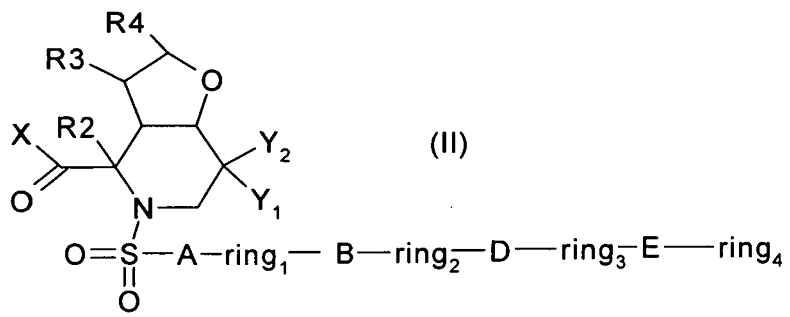

(II)

A für eine kovalente Bindung steht,
B, D und E gleich oder verschieden sind und unabhängig voneinander für eine kovalente Bindung oder den Rest -O- stehen,
ring 1, ring2 oder ring3 gleich oder verschieden sind und unabhängig voneinander für

1) kovalente Bindung stehen oder
2) Phenyl bedeutet und unsubstituiert oder unabhängig voneinander ein- oder zweifach durch G substituiert sind,

mit der Maßgabe, dass mindestens einer der Reste ring 1, ring2 oder ring3 für Phenyl steht,
ring4 für Phenyl steht und unsubstituiert oder unabhängig voneinander ein- oder zweifach durch G substituiert ist,
G für 1) Wasserstoffatom,

2) Br, Cl oder F,
3) -($C_1$-$C_4$)-Alkyl, worin Alkyl unsubstituiert oder ein-, zwei- oder dreifach durch Br, Cl oder F substituiert ist,

4) -$SO_2$-Methyl oder
5) -CN steht,

X für -OH oder-NH-OH steht, und
R2, R3, R4, Y1 und Y2 gleich sind und für Wasserstoffatom stehen.

**2.** Verbindung der Formel II gemäß Anspruch 1, **dadurch gekennzeichnet, dass** es die Verbindung
5-(4'-Chlorbiphenyl-4-sulfonyl)-octahydrofuro [3,2-c] pyridin-4-carbonsäure,
5-(4'-Chlorbiphenyl-4-sulfonyl)-octahydrofuro[3,2-c]pyridin-4-(N-hydroxy)-carboxamid,
5-[4-(4-Fluorphenoxy)-benzolsulfonyl]-octahydro-furo[3,2-c]pyridin-4-carbonsäure,
5-[4-(4-Fluorphenoxy)-benzolsulfonyl]-octahydro-furo[3,2-c]pyridin-4-(N-hydroxy)-carboxamid,
5-[4-(4-Cyanophenoxy)-benzolsulfonyl]-octahydro-furo[3,2-c]pyridin-4-carbonsäure,
5-[4-(4-Methansulfonylphenoxy)-benzolsulfonyl]-octahydro-furo[3,2-c] pyridin-4-carbonsäure,
5-(4'-Fluorbiphenyl-4-sulfonyl)-octahydro-furo[3,2-c] pyridin-4-(N-hydroxy)-carboxamid,
5-(4'-Trifluormethylbiphenyl-4-sulfonyl)-octahydro-furo[3,2-c]pyridin-4-(N-hydroxy)-carboxamid,
5-[4-(4-Chlorphenoxy)-benzolsulfonyl]-octahydro-furo [3,2-c] pyridin-4-(N-hydroxy)-carboxamid,
5-[4-(4-Cyanophenoxy)-benzolsulfonyl]-octahydro-furo [3,2-c] pyridin-4-(N-hydroxy)-carboxamid oder
5-[4-(4-Methansulfonylphenoxy)-benzolsulfonyl]-octahydro-furo[3,2-c]pyridin-4-(N-hydroxy) carboxamid ist.

**3.** Verfahren zur Herstellung der Verbindung der Formel II gemäß der Ansprüche 1 oder 2, **dadurch gekennzeichnet, dass** man

a) eine Verbindung der Formel IV,

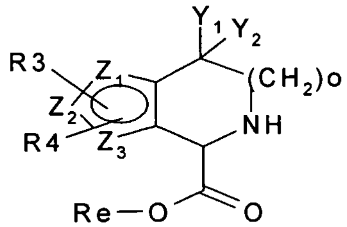

(IV)

worin Re ein Wasserstoffatom oder eine Ester-Schutzgruppe darstellt, die Reste Y 1, Y2, R3, R4 und o wie in der Verbindung der Formel II definiert sind und die Teilstruktur der Verbindung der Formel II

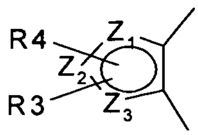

ein ungesättigter Ring mit 5 Ringatomen ist, wobei eines der Ringatome Z1, Z2 oder Z3 für ein Sauerstoffatom steht und die beiden anderen Ringatome für Kohlenstoffatome stehen, die unabhängig voneinander durch R3 oder R4 substituiert sind,
durch Hydrierung unter geeigneten Bedingungen zu einer Verbindung der Formel V umwandelt,

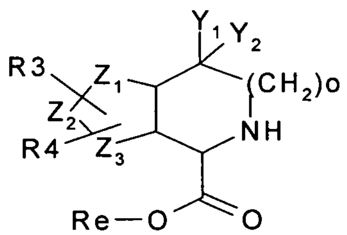

(V)

worin die Teilstruktur der Verbindung der Formel II

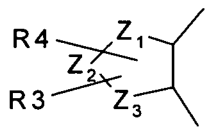

ein gesättigter Ring mit 5 Ringatomen ist, wobei eines der Ringatome Z1, Z2 oder Z3 für ein Sauerstoffatom steht und die beiden anderen Ringatome für Kohlenstoffatome stehen, die unabhängig voneinander durch R3 oder R4 substituiert sind, und die Reste Y1, Y2, R3, R4 und o wie in der Verbindung der Formel IV definiert sind,
b) anschließend die Verbindung der Formel V mit einer Verbindung der Formel VI,

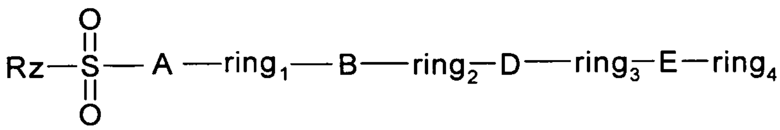

(VI)

worin A, B, D, E und ring1, ring2, ring3, ring4 wie in Formel II definiert sind, und worin Rz Chloratom, Bromatom, Imidazoyl oder OH bedeutet,
in Gegenwart einer Base oder nach Silylierung mit einem geeigneten Silylierungsmittel oder mit einem geeigneten wasserentziehenden Mittel für den Fall Rz = OH zu einer Verbindung der Formel VII umsetzt,

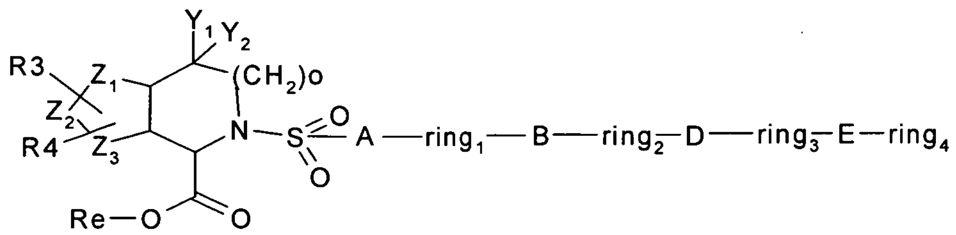

**(VII)**

worin A, B, D, E, Re und ring1, ring2, ring3 und ring4 wie oben definiert sind, und
b) für den Fall Re = Ester eine nach a) hergestellte Verbindung der Formel VII mit einer Alkalilauge wie NaOH oder LiOH und anschließender Säurebehandlung zu der erfindungsgemäßen Carbonsäure der Formel II, worin X = OH ist, umsetzt, wobei gegebenenfalls vorher noch Modifikationen in einer der Seitenketten der Ringe ring1-ring4 vorgenommen wurden; oder den genannten Ester durch Behandlung mit Mineralsäure wie Salzsäure zur freien Carbonsäure der Formel VIII umsetzt

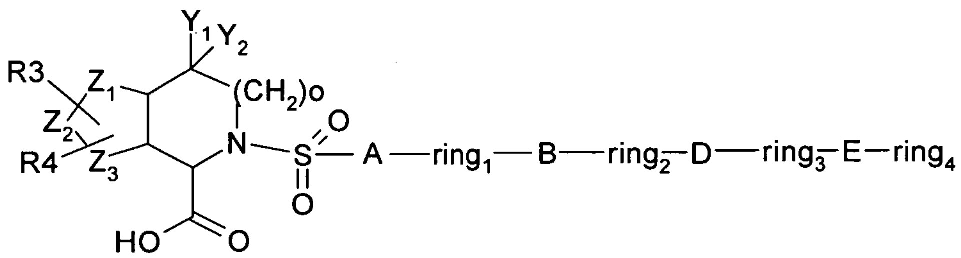

(VIII)

oder anschließend die Verbindung der Formel VIII in die Hydroxamsäure, worin X = NH-OH ist, der Formel II umwandelt,
c) eine nach Verfahren a) hergestellte Verbindung der Formel II, oder eine geeignete Vorstufe der Formel II, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder
d) die nach den Verfahren b) oder c) hergestellte Verbindung der Formel II entweder in freier Form isoliert oder im Falle des Vorliegens von sauren oder basischen Gruppen in physiologisch verträgliche Salze umwandelt.

4. Arzneimittel, **gekennzeichnet durch** einen wirksamen Gehalt an mindestens einer Verbindung der Formel II gemäß der Ansprüche 1 oder 2 zusammen mit einem pharmazeutisch geeigneten und physiologisch verträglichen Trägerstoff, Zusatzstoff und/oder anderen Wirk- und Hilfsstoffen.

5. Verwendung der Verbindung der Formel II gemäß der Ansprüche 1 oder 2 zur Herstellung eines Arzneimittels zur Prophylaxe und Therapie von degenerativen Gelenkerkrankungen wie Osteoarthrosen, Spondylosen, Knorpelschwund nach Gelenktrauma oder längerer Gelenksruhigstellung nach Meniskus- oder Patellaverletzungen oder Bänderrissen, Erkrankungen des Bindegewebes wie Kollagenosen, Periodontalerkrankungen, Wundheilungsstörungen und chronische Erkrankungen des Bewegungsapparates wie entzündliche, immunologisch oder stoffwechselbedingte akute und chronische Arthritiden, Arthropathien, Myalgien und Störungen des Knochenstoffwechsels, zur Behandlung der Ulceration, Atherosklerose und Stenosen, Behandlung von Entzündungen, Krebserkrankungen, Tumormetastasenbildung, Kachexie, Anorexie, Herzversagen und septischem Schock oder Prophylaxe von Myocard- und Cerebral-Infarkten.

## Claims

1. A compound of the formula II, where

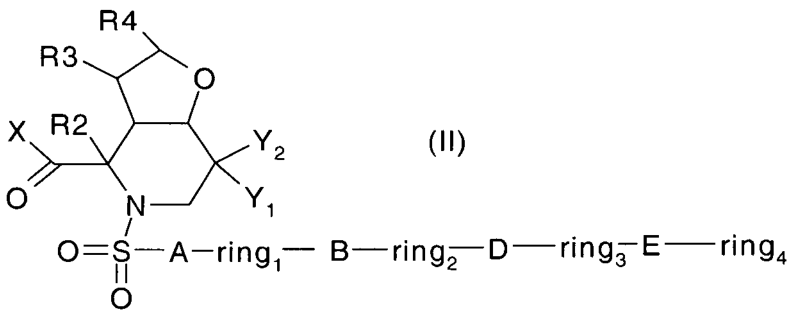

(II)

A is a covalent bond,
B, D and E are identical or different and are independently of one another a covalent bond or the radical -O-,
ring1, ring2 or ring3 are identical or different and are independently of one another

1) covalent bond, or
2) is phenyl and are unsubstituted or substituted independently of one another once or twice by G,

with the proviso that at least one of the radicals ring1, ring2 or ring3 is phenyl,
ring4 is phenyl and is unsubstituted or substituted independently of one another once or twice by G,
G is 1) hydrogen atom,

2) Br, C1 or F,
3) -($C_1$-$C_4$)-alkyl in which alkyl is unsubstituted or substituted once, twice or three times by Br, Cl or F,
4) -$SO_2$-methyl, or
5) -CN,

X is -OH or -NH-OH, and
R2, R3, R4, Y1 and Y2 are identical and are hydrogen atom.

2. A compound of the formula II as claimed in claim 1, which is the compound
5-(4'-chlorobiphenyl-4-sulfonyl)octahydrofuro[3,2-c]pyridine-4-carboxylic acid,
5-(4'-chlorobiphenyl-4-sulfonyl)octahydrofuro[3,2-c]pyridine-4-(N-hydroxy)-carboxamide,
5-[4-(4-fluorophenoxy)benzenesulfonyl]octahydrofuro[3,2-c]pyridine-4-carboxylic acid,
5-[4-(4-fluorophenoxy)benzenesulfonyl]octahydrofuro[3,2-c]pyridine-4-(N-hydroxy)-carboxamide,
5-[4-(4-cyanophenoxy)benzenesulfonyl]octahydrofuro[3,2-c]pyridine-4-carboxylic acid,
5-[4-(4-methanesulfonylphenoxy)benzenesulfonyl]octahydrofu ro[3,2-c]pyridine-4-carboxylic acid,
5-(4'-fluorobiphenyl-4-sulfonyl)octahydrofuro[3,2-c]pyridine-4-(N-hydroxy)-carboxamide,

5-(4'-trifluoromethylbiphenyl-4-sulfonyl)octahydrofuro[3,2-c]pyridine-4-(N-hydroxy)-carboxamide,
5-[4-(4-chlorophenoxy)benzenesulfonyl]octahydrofuro[3,2-c]pyridine-4-(N-hydroxy)-carboxamide,
5-[4-(4-cyanophenoxy)benzenesulfonyl]octahydrofuro[3,2-c]pyridine-4-(N-hydroxy)-carboxamide or
5-[4-(4-methanesulfonylphenoxy)benzenesulfonyl]octahydrofu ro[3,2-c]pyridine-4-(N-hydroxy)carboxamide.

**3.** A method for preparing the compound of the formula II as claimed in claim 1 or 2, which comprises

a) a compound of the formula IV

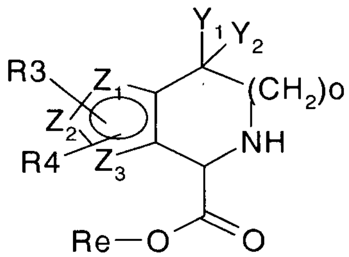

**(IV)**

in which Re is a hydrogen atom or an ester protective group, the radicals Y1, Y2, R3, R4 and o are as defined in the compound of the formula II, and the partial structure of the compound of the formula II

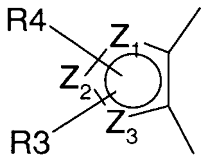

is an unsaturated ring having 5 ring atoms, where one of the ring atoms Z1, Z2 or Z3 is an oxygen atom, and the two other ring atoms are carbon atoms which are substituted independently of one another by R3 or R4, being converted by hydrogenation under suitable conditions into a compound of the formula V

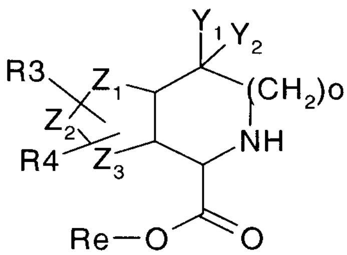

**(V)**

in which the partial structure of the compound of the formula II

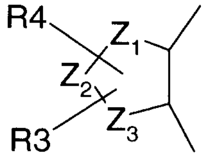

is a saturated ring having 5 ring atoms, where one of the ring atoms Z1, Z2 or Z3 is an oxygen atom, and the two other ring atoms are carbon atoms which are substituted independently of one another by R3 or R4, and the radicals Y1, Y2, R3, R4 and o are as defined in the compound of the formula IV,

b) subsequently the compound of the formula V being reacted with a compound of the formula VI

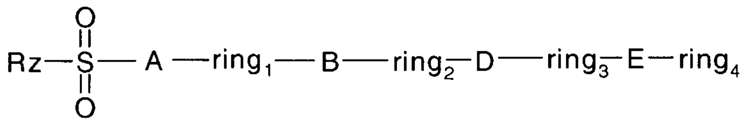

(VI)

in which A, B, D, E and ring1, ring2, ring3, ring4 are as defined in formula II, and in which Rz is chlorine atom, bromine atom, imidazolyl or OH,
in the presence of a base or after silylation with a suitable silylating agent or with a suitable dehydrating agent in the case where Rz = OH to give a compound of the formula VII

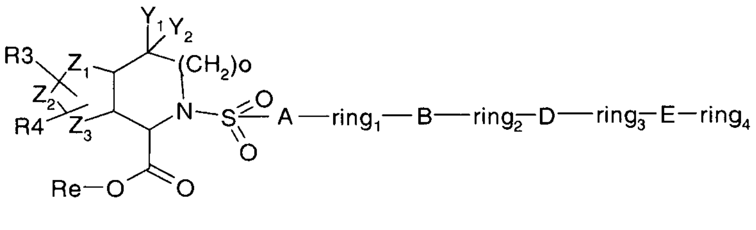

(VII)

in which A, B, D, E, Re and ring1, ring2, ring3 and ring4 are as defined above, and
b) in the case where Re = ester, a compound of the formula VII prepared as in a) being reacted with an alkali metal hydroxide solution such as NaOH or LiOH and subsequent acid treatment to give the carboxylic acid of the invention of the formula II in which X is OH, with modifications in one of the side chains of the rings ringl-ring4 having been undertaken where appropriate beforehand; or said ester being converted by treatment with mineral acids such as hydrochloric acid into the free carboxylic acid of the formula VIII

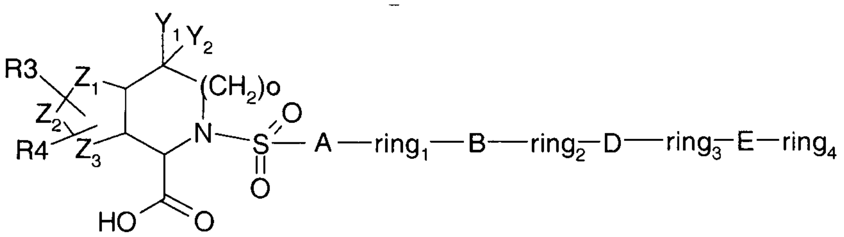

(VIII)

or subsequently the compound of the formula VIII being converted into the hydroxamic acid in which X is NH-OH, of the formula II,
c) a compound of the formula II prepared by method a), or a suitable precursor of the formula II which, owing to its chemical structure, occurs in enantiomeric forms, being fractionated into the pure enantiomers by salt formation with enantiopure acids or bases, chromatography on chiral stationary phases or derivatization with chiral enantiopure compounds such as amino acids, separation of the diastereomers obtained in this way, and elimination of the chiral auxiliary groups, or
d) the compound of the formula II prepared by methods b) or c) either being isolated in free form or, in the case where acidic or basic groups are present, being converted into physiologically tolerated salts.

**4.** A medicament having an effective content of at least one compound of the formula II as claimed in claim 1 or 2 together with a pharmaceutically suitable and physiologically tolerated carrier, additive and/or other active ingredients and excipients.

**5.** The use of the compound of the formula II as claimed in claim 1 or 2 for producing a medicament for the prophylaxis and therapy of degenerative joint disorders such as osteoarthroses, spondyloses, chondrolysis following joint trauma or prolonged joint immobilization after meniscus or patella injuries or ligament tears, connective tissue disorders such as collagenoses, periodontal disorders, wound-healing disturbances and chronic disorders of the locomotor system such as inflammatory, immunologically or metabolically related acute and chronic arthritides, arthropathies, myalgias and disturbances of bone metabolism, for the treatment of ulceration, atherosclerosis and stenoses, treatment of inflammations, cancers, tumor metastasis, cachexia, anorexia, heart failure and septic shock or prophylaxis of myocardial and cerebral infarctions.

## Revendications

**1.** Composé de formule II,

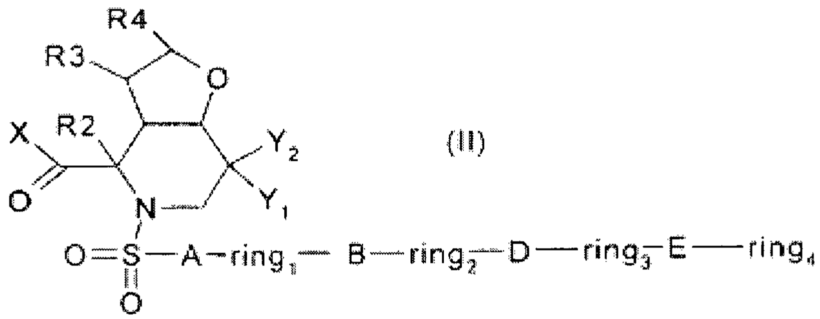

(II)

dans laquelle

A représente une liaison covalente,
B, D et E sont identiques ou différents et représentent indépendamment l'un de l'autre une liaison covalente ou le radical -O-,
ring1, ring2 et ring3 sont identiques ou différents et chacun indépendamment

1) représentent une liaison covalente ou
2) représentent un groupe phényle et sont non substitués ou une ou deux fois substitués chacun indépendamment par G,

étant entendu qu'au moins l'un des radicaux ring1, ring2 ou ring3 représente phényle,
ring4 représente un groupe phényle et est non substitué ou une ou deux fois indépendamment l'une de l'autre par G,
G représente 1) un atome d'hydrogène,

2) Br, Cl ou F,
3) un groupe -alkyle en $C_1$-$C_4$, le groupe alkyle étant non substitué ou une, deux ou trois fois substitué par Br, C1 ou F,
4) -$SO_2$-méthyle ou
5) -CN,

X représente -OH ou -NH-OH, et
R2, R3, R4, Y1 et Y2 sont identiques et représentent un atome d'hydrogène.

**2.** Composé de formule II selon la revendication 1, **caractérisé en ce qu'**il est le composé
acide 5-(4'-chlorobiphényl-4-sulfonyl)-octahydrofuro[3,2-c]pyridine-4-carboxylique,
5-(4'-chlorobiphényl-4-sulfonyl)-octahydrofuro[3,2-c]-pyridine-4-(N-hydroxy)-carboxamide,
acide 5-[4-(4-fluorophénoxy)-benzènesulfonyl]-octahydro-furo[3,2-c]pyridine-4-carboxylique,
5-[4-(4-fluorophénoxy)-benzènesulfonyl]-octahydro-furo[3,2-c]pyridine-4-(N-hydroxy)-carboxamide,
acide 5-[4-(4-cyanophénoxy)-benzènesulfonyl]-octahydro-furo[3,2-c]pyridine-4-carboxylique,
acide 5-[4-(4-méthanesulfonylphénoxy)-benzènesulfonyl]-octahydro-furo[3,2-c]pyridine-4-carboxylique,
5-(4'-fluorobiphényl-4-sulfonyl)-octahydro-furo[3,2-c]-pyridine-4-(N-hydroxy)-carboxamide,
5-(4'-trifluorométhylbiphényl-4-sulfonyl)-octahydro-furo[3,2-c]pyridine-4-(N-hydroxy)-carboxamide,
5-[4-(4-chlorophénoxy)-benzènesulfonyl]-octahydro-furo[3,2-c]pyridine-4-(N-hydroxy)-carboxamide,
5-[4-(4-cyanophénoxy)-benzènesulfonyl]-octahydro-furo[3,2-c]pyridine-4-(N-hydroxy)-carboxamide ou
5-[4-(4-méthanesulfonylphénoxy)-benzènesulfonyl]-octahydro-furo[3,2-c]pyridine-4-(N-hydroxy)-carboxamide.

**3.** Procédé pour la préparation du composé de formule II selon la revendication 1 ou 2, **caractérisé en ce que**

a) on convertit un composé de formule IV,

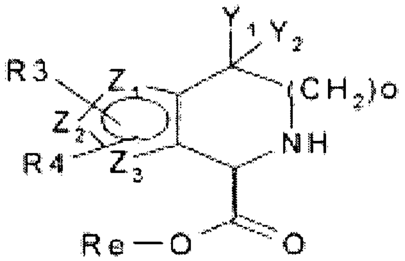

(IV)

dans laquelle Re représente un atome d'hydrogène ou un groupe protecteur d'ester, les radicaux Y1, Y2, R3, R4 et o sont tels que définis dans le composé de formule II et la structure partielle du composé de formule II

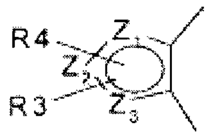

est un cycle insaturé ayant 5 atomes formant le cycle, l'un des atomes formant le cycle Z1, Z2 ou Z3 représentant un atome d'oxygène et les deux autres atomes formant le cycle représentant des atomes de carbone qui sont substitués indépendamment l'un de l'autre par R3 ou R4,
par hydrogénation dans des conditions convenables, en un composé de formule V,

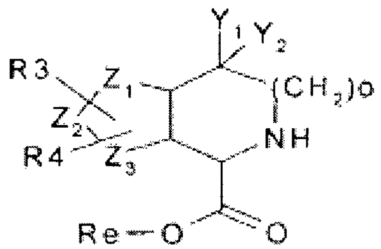

(V)

dans laquelle la structure partielle du composé de formule II

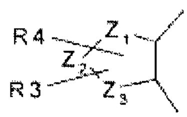

est un cycle saturé ayant 5 atomes formant le cycle, l'un des atomes formant le cycle Z1, Z2 ou Z3 représentant un atome d'oxygène et les deux autres atomes formant le cycle représentant des atomes de carbone qui sont substitués indépendamment l'un de l'autre par R3 ou R4, et les radicaux Y1, Y2, R3, R4 et o sont tels que définis dans le composé de formule IV, b) ensuite on fait réagir le composé de formule V avec un composé de formule VI,

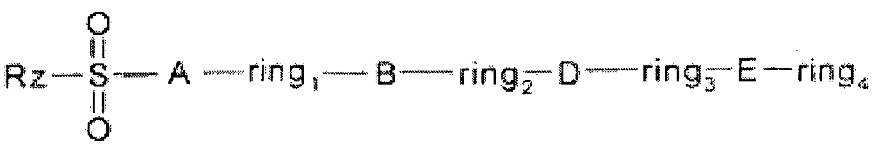

(VI)

dans laquelle A, B, D, E et ring1, ring2, ring3, ring4 sont tels que définis dans la formule II, et dans laquelle Rz représente un atome de chlore, un atome de brome, le groupe imidazolyle ou OH,
en présence d'une base ou après silylation à l'aide d'un agent de silylation approprié ou avec un agent déshydratant approprié dans le cas où Rz = OH, pour obtenir un composé de formule VII,

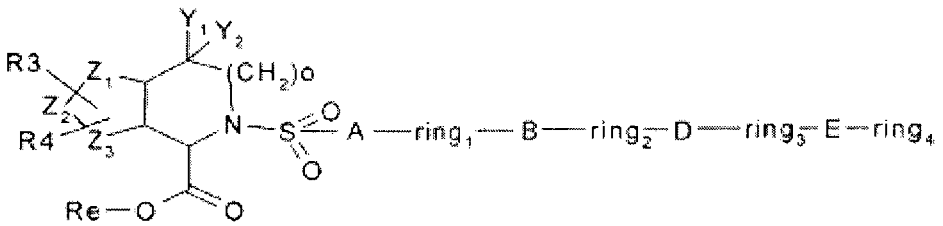

(VII)

dans laquelle A, B, D, E, Re et ring1, ring2, ring3 et ring4 sont tels que définis plus haut, et

b) dans le cas où Re = ester, on fait réagir un composé de formule VII préparé selon a) avec une solution d'un hydroxyde de métal alcalin tel que NaOH ou LiOH et traitement subséquent par un acide, pour aboutir à l'acide carboxylique de formule II selon l'invention, dans laquelle X = OH, en ayant éventuellement effectué au préalable des modifications dans l'une des chaînes latérales des cycles ringl-ring4 ; ou on convertit ledit ester, par traitement par un acide minéral tel que l'acide chlorhydrique, en l'acide carboxylique libre de formule VIII

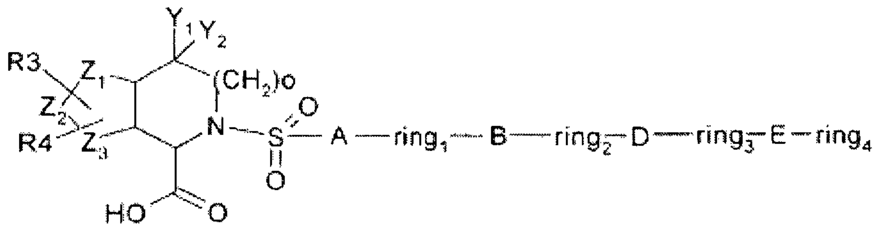

(VIII)

ou ensuite on convertit le composé de formule VIII en l'acide hydroxamique, dans lequel X = NH-OH, de formule II,

c) on résout en les énantiomères purs un composé de formule II, préparé selon le procédé a), ou un précurseur approprié de formule II, qui en raison de sa structure chimique apparaît sous des formes énantiomères, par salification avec des acides ou des bases sous forme d'énantiomères purs, chromatographie sur des phases stationnaires chirales ou transformation en dérivés à l'aide de composés chiraux sous forme d'énantiomères purs, tels que des acides aminés, séparation des diastéréoisomères ainsi obtenus, et élimination des groupes auxiliaires chiraux, ou

d) soit on isole sous forme libre le composé de formule II préparé selon le procédé b) ou c), soit, dans le cas de la présence de groupes acides ou basiques, on le convertit en sels physiologiquement acceptables.

4. Médicament, **caractérisé par** une teneur efficace en au moins un composé de formule II selon la revendication 1 ou 2, conjointement avec un véhicule, un additif, pharmaceutiquement appropriés et physiologiquement acceptables et/ou d'autres substances actives et adjuvants.

5. Utilisation du composé de formule II selon la revendication 1 ou 2 pour la fabrication d'un médicament destiné à la prophylaxie et la thérapie d'arthropathies dégénératives telles que des ostéo-arthroses, des spondyloses, une chondroporose après traumatisme articulaire ou relativement longue immobilisation d'articulations après lésions du ménisque ou de la rotule ou déchirures de ligaments, de maladies du tissu conjonctif telles que les collagénoses, les maladies parodontales, les troubles de la cicatrisation et de maladies chroniques de l'appareil moteur, telles que les arthrites, arthropathies, myalgies, aiguës et chroniques, d'origine inflammatoire, immunologique ou métabolique, et de troubles du métabolisme du tissu osseux, au traitement de l'ulcération, de l'athérosclérose et de sténoses, au traitement d'inflammations, de maladies cancéreuses, de la formation de métastases tumorales, de la cachexie, de l'anorexie, de l'insuffisance cardiaque et du choc septique ou à la prophylaxie d'infarctus du myocarde et cérébraux.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0606046 A
- WO 9428889 A
- WO 9627583 A
- EP 0803505 A
- EP 1065209 A
- EP 1217002 A
- WO 9906410 A
- US 0226018 W
- WO 2002100860 A
- WO 9718194 A


### In der Beschreibung aufgeführte Nicht-Patentliteratur


- **D. YIP et al.** *Investigational New Drugs,* 1999, vol. 17, 387-399
- **MICHAELIDES et al.** *Current Pharmaceutical Design,* 1999, vol. 5, 787-819
- **S. J. GEORGE.** *Exp. Opin. Invest. Drugs,* 2000, vol. 9 (5), 993-1007
- **E. J. M. CREEMER et al.** *Circulation Res.,* 2001, vol. 89, 201-210
- *Drugs,* 2001, vol. 61, 1239-1252
- *Current Medicinal Chemistry,* 2001, vol. 8, 425-74
- *Current Medicinal Chemistry,* 2004, vol. 11, 2911-2977
- *Current Opinion in Drug Discovery & Development,* 2004, vol. 7, 513-535
- **MASSOVA I et al.** *The FASEB Journal,* 1998, vol. 12, 1075-1095
- *Science of Synthesis,* 2002, vol. 9, 183-285
- *J. Med. Chem.,* 1994, vol. 37, 2138-2144
- *Arch. Pharm.,* 2003, vol. 336, 381-4
- *Synthesis,* 2004, 2069-2077
- *Heterocycles,* 1993, vol. 35, 737-754
- *J. Heterocycl. Chem.,* 2000, vol. 37, 751-55
- *Synth. Commun.,* 1995, vol. 25, 2895-2900
- *J. Mol. Catal.,* 1990, vol. 57, 397
- *J. Heterocycl. Chem.,* 1966, vol. 3, 101
- *J. Org. Chem.,* 1972, vol. 37, 4260
- *Org. Synth.,* 1943, vol. II, 566
- *J. Chem. Soc.,* 1957, 1788
- *Monatsh. Chem.,* 2000, vol. 131, 1335-1343
- *Chem. Lett.,* 1999, 1055-56
- *Progress in Heterocyclic Chemistry,* 2002, vol. 14, 139
- *Progress in Heterocyclic Chemistry,* 1995, vol. 7, 130
- **T.H. GREENE ; P. G. M. WUTS.** Protective Groups in Organic Synthesis. Wiley-Interscience, 1999
- **YE et al.** *Biochemistry,* 1992, vol. 31, 11231-11235